(19) Europäisches Patentamt
European Patent Office
Office européen des brevets

(11) EP 4 566 606 A1

(12) EUROPEAN PATENT APPLICATION
published in accordance with Art. 153(4) EPC

(43) Date of publication:
**11.06.2025 Bulletin 2025/24**

(21) Application number: **23850147.2**

(22) Date of filing: **03.08.2023**

(51) International Patent Classification (IPC):
***A61K 31/4425*** (2006.01) ***A61K 9/14*** (2006.01)
***A61K 9/51*** (2006.01) ***A61K 47/24*** (2006.01)
***A61L 9/16*** (2006.01) ***A61L 27/28*** (2006.01)
***A61L 27/54*** (2006.01) ***A61L 29/08*** (2006.01)
***A61L 29/16*** (2006.01) ***A61L 31/08*** (2006.01)
***A61L 31/16*** (2006.01) ***A61P 31/00*** (2006.01)
***B01D 39/16*** (2006.01) ***B01D 39/18*** (2006.01)
***D06M 13/463*** (2006.01)

(52) Cooperative Patent Classification (CPC):
**A61K 9/14; A61K 9/51; A61K 31/4425; A61K 47/24; A61L 9/16; A61L 27/28; A61L 27/54; A61L 29/08; A61L 29/16; A61L 31/08; A61L 31/16; A61P 31/00; B01D 39/16; B01D 39/18; D06M 13/463**

(86) International application number:
**PCT/JP2023/028430**

(87) International publication number:
**WO 2024/029594 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.08.2022 JP 2022125464**
**19.01.2023 JP 2023006898**

(71) Applicant: **Amorufus Co., Ltd.**
**Osaka-shi, Osaka 541-0046 (JP)**

(72) Inventors:
- **ISHIKAWA, Hiroshi**
  **Otsu-shi, Shiga 520-0023 (JP)**
- **KISHIHARA, Tadao**
  **Nishinomiya-shi, Hyogo 662-0033 (JP)**
- **MURATA, Koichiro**
  **Kawanishi-shi, Hyogo 666-0152 (JP)**

(74) Representative: **Gilani, Anwar et al**
**Venner Shipley LLP**
**406 Science Park**
**Milton Road**
**Cambridge CB4 0WW (GB)**

# (54) CETYLPYRIDINIUM CHLORIDE HYDRATE-CONTAINING SILICON NANOPARTICLE COMPOSITION AND TECHNOLOGY OF APPLYING SAME

(57) A specific silicon compound is represented by the following formula:

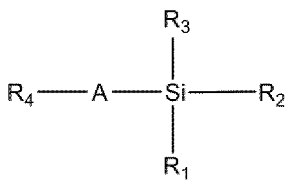

wherein each symbol is as defined in the specification. A composition comprises the silicon compound and cetylpyridinium chloride hydrate and is in the form of nanoparticles. The composition can be easily attached to or carried by a medical material or a medical device in a favorable manner.

EP 4 566 606 A1

## Description

### Technical Field

The present invention relates to a composition comprising cetylpyridinium chloride hydrate and a silicon compound that is for preventing and/or treating an infectious disease and contains a surfactant that is effective against enveloped viruses in general, including the novel coronavirus (SARS-Co-2). The present invention also relates to a medical material carrying the composition, and a novel drug delivery system using the medical material or medical device.

### Background Art

Cetylpyridinium chloride hydrate is a quaternary ammonium compound having a pyridine ring and has strong bactericidal action against gram-positive bacteria including staphylococci, as well as other fungi. Due to its properties, cetylpyridinium chloride hydrate is used in dentifrices, troches for bacterial killing in the mouth, or pharmaceutical products, such as mouthwashes for bacterial killing in the throat (Wikipedia).

Furthermore, cetylpyridinium chloride hydrate is known to be effective against enveloped viruses in general, including the novel coronavirus (SARS-Co-2) (Non-patent Literature (NPL) 1).

As of July 16, 2022, COVID-19 is a pandemic that has shaken the world with no end in sight, in which more than 560 million people have been infected worldwide, and more than 6 million people have died. Although a number of therapeutic agents have been developed, the primary measure is vaccination, and as supplementary preventive measures, wearing masks, washing hands, and avoiding closed spaces, crowded places, and close contact settings are widely recommended.

These behaviors, except for hand-washing, correspond to avoiding the risk of infection through inhalation of droplets containing the virus. Simulations have revealed the effect of humidity on the spread of droplets and the effect of masks, and droplet infection is positioned as the main route of infection.

On the other hand, the time for which the virus attached to a material surface has infectious activity depends heavily on temperature and humidity, and it has been reported that the virus remains infectious for 1 week at low temperatures and low humidity. Thus, virus inactivation on solid surfaces remains an important issue (NPL 2 and NPL 3).

Alcohols and surfactants can disrupt the envelopes of enveloped viruses, including the novel coronavirus, and their effects do not change even if the virus surface proteins are mutated. Thus, they are also expected to be effective against variant viruses. Wiping with a surfactant is effective; however, it is difficult to do so frequently on objects that people touch with their hands in public places, such as doorknobs, handrails, and straps. These require inactivation for a short period of time and persistence of the antiviral effect.

There is also the problem of prosthetic joint infections. In general, as the number of elderly people increases, the number of fracture patients and the number of surgical operations associated with fractures are expected to increase. In recent years, bone and joint surgery using prosthetic joints has been on the rise, and it has been reported that the number of patients in the U.S. will reach 4 million per year by 2030. However, in surgery using prosthetic joints, prosthetic joint infections (PJI) may occur after surgery.

Prosthetic joint infections occur in about 1% of patients in Japan and in about 1 to 2% of patients in the U.S. Many patients who develop prosthetic joint infections require multiple surgical operations, which places a significant burden on the patients, and may require amputation of the surgical site, which in the worst-case scenario can lead to death.

Since prosthetic joint infections are generally difficult to diagnose and treat by drug administration, antimicrobial agent- or antibiotic-containing sutures or cements are sometimes used. However, their effects are not clear, and the treatment cost is high.

In addition to prosthetic joint infections, postoperative infections, such as catheter-related bloodstream infections caused by intra-body catheters and infectious diseases in the urinary tract, biliary tract, etc. are also problems.

Under such circumstances, in the medical settings of orthopedics, surgery, urology, etc., medical materials containing, for example, an antimicrobial agent or a bactericidal agent in which the effect of such an agent is sustained are desired to prevent postoperative infectious diseases caused by a medical material or a medical device. There have been attempts to treat or prevent infectious diseases or the like by allowing a medicinal component to be carried by a medical material or a medical device by using a silicon compound (Patent Literature (PTL) 1 to PTL 4).

However, none of the compositions described in these documents is capable of sterile filtration, and none of these documents discloses that a medicinal component is carried by a medical material or a medical device by using a silicon compound in combination with cetylpyridinium chloride hydrate to treat or prevent an infectious disease or the like by a sustained antimicrobial and/or bactericidal effect. NPL 4 shows a composition containing a drug and a silicon compound.

### Citation List

Non-patent Literature

NPL 1: https://specchem-wako-jp.fujifilm.com/cpc/
NPL 2: C. Xie, H. Zao, K. Li, Z. Zhang, X. Lu, H. Pang, D. Wang, J. Chen, X. Zhang, D. Wu, Y. Gu, Y. Yuan, L. Zhang, and J. Lu, The evidence of indirect transmission of SARS-CoV-2 reported in Guangzhou, China BMC Public Health, 20. 2020, 1202.
NPL 3: S. H. Bae, H. Shin, H-Y. Koo, S. W. Lee, J. M. Yang, and D. K. Yon, Asymptomatic transmission of SARS-Co-2 on evacuation flight, Emerg. Infect, Dis., 26, 2020, 2705.
NPL 4: Journal of American Chemical Society, Vol. 125, No. 26, 2003, pp. 7860-7865

Patent Literature

PTL 1: JP2007-505697A
PTL 2: JP2008-266157A
PTL 3: JP2008-506493A
PTL 4: JP2012-533568A

Summary of Invention

Technical Problem

One problem to be solved by the present invention is to provide a novel virus detection and capture device by allowing cetylpyridinium chloride hydrate to be carried by sanitary materials, such as masks, gloves, gauze, and surgical tape, or various preventive shielding plates as a measure against COVID-19 to use the effect of preventing the infection by the viruses that come into contact with water.
Another problem is to provide a novel drug delivery system in which cetylpyridinium chloride hydrate is stably carried by a medical material or a medical device, and an antimicrobial and/or bactericidal effect is exhibited in a sustained manner after the medical material or medical device is placed in the body.

Solution to Problem

As a result of extensive research, the present inventors prepared a nanoparticle composition containing cetylpyridinium chloride hydrate in a specific silicon compound, and found that the above problems are solved by the composition. The present invention has thus been accomplished. That is, the basic idea of the present invention lies in a composition that comprises cetylpyridinium chloride hydrate and a specific silicon compound, and that is in the form of nanoparticles. The nanoparticle form exhibits high adhesion or carrying properties for medical devices and the like, and also enables sterile filtration required for medical devices.
The present invention includes the embodiments described below.

Item 1.

A compound represented by formula 1 or a salt thereof,

Formula 1

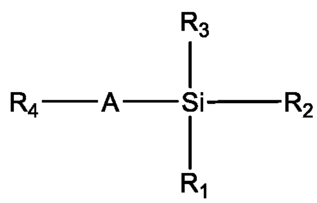

wherein

A represents a bond or lower alkylene;
$R_1$, $R_2$, and $R_3$ each independently represent

(1) lower alkyl,
(2) O-$R_5$, or
(3) phenyl;

$R_5$ represents

(1) lower alkyl or
(2) lower alkanoyl;

$R_4$ represents

(1) lower alkyl,
(2) lower alkenyl,
(3) phenyl,
(4) amino optionally substituted with one to two identical or different $R_6$s,
(5) $R_5$-CONH, or
(6) cyano;

$R_6$ represents

(1) lower alkyl optionally substituted with one to two identical or different $R_7$s,
(2) amino optionally substituted with one to two identical or different $R_8$s, or
(3) silyl lower alkyl optionally substituted with one to three identical or different lower alkoxy groups

$R_7$ represents

(1) amino optionally substituted with one to two identical or different lower alkyl groups,
(2) phenyl optionally substituted with hydroxy,
(3) carbamoyl,
(4) imidazolyl optionally substituted with benzyl, or
(5) carboxy, and

$R_8$ represents

(1) amino lower alkyl, wherein the amino is optionally substituted with one to two identical or different lower alkyl groups, or

two $R_8$s together with the nitrogen to which they are attached may form a monocyclic or bicyclic heterocycle, wherein the compound of formula (I) may be two or more different compounds represented by formula (I).

Item 2.

The composition according to Item 1, wherein the compound of formula (I) is a compound in which

A represents a bond or $C_{1-6}$ alkylene;
$R_1$, $R_2$, and $R_3$ each independently represent

(1) $C_{1-6}$ alkyl,
(2) O-$R_5$, or
(3) phenyl;

$R_5$ represents

(1) $C_{1-6}$ alkyl or
(2) $C_{1-6}$ alkanoyl,

$R_4$ represents

(1) $C_{1-8}$ alkyl
(2) $C_{2-4}$ alkenyl,
(3) phenyl,
(4) amino optionally substituted with one $R_6$,
(5) $R_6$-CONH, or
(6) cyano;

$R_6$ represents

(1) $C_{1-6}$ alkyl optionally substituted with one to two identical or different $R_7$s,
(2) amino optionally substituted with one to two identical or different $R_8$s, or
(3) silyl $C_{1-6}$ alkyl optionally substituted with one to three identical or different $C_{1-6}$ alkoxy groups;

$R_7$ represents

(1) amino optionally substituted with one to two identical or different $C_{1-6}$ alkyl groups,
(2) phenyl substituted with hydroxy,
(3) carbamoyl,
(4) benzylimidazolyl, or
(5) carboxy; and

$R_8$ represents amino-$C_{1-6}$ alkyl, wherein the amino is optionally substituted with one to two identical or different $C_{1-6}$ alkyl groups; or two $R_8$s together with the nitrogen to which they are attached may form a saturated or unsaturated 3- to 12-membered monocyclic or bicyclic heterocycle containing at least one nitrogen as a ring-constituting heteroatom.

Item 3.

The composition according to Item 1 or 2, wherein the compound of formula (I) is a compound in which $R_4$ is $R_6$-CONH.

Item 4.

The composition according to any one of Items 1 to 3, wherein the compound of formula (I) is a compound in which

A represents $C_{1-6}$ alkylene;
$R_1$, $R_2$, and $R_3$ each independently represent O-$R_5$;
$R_5$ represents $C_{1-6}$ alkyl;
$R_4$ represents $R_6$-CONH;
$R_6$ represents

(1) $C_{1-6}$ alkyl optionally substituted with one to two identical or different $R_7$s, or
(2) amino optionally substituted with one to two identical or different $R_8$s;

$R_7$ represents

(1) amino optionally substituted with two identical or different $C_{1-6}$ alkyl groups,
(2) phenyl substituted with hydroxy,
(3) carbamoyl,
(4) benzylimidazolyl, or
(5) carboxy, and

$R_8$ represents amino-$C_{1-6}$ alkyl, wherein the amino is optionally substituted with one to two identical or different $C_{1-6}$ alkyl groups.

Item 5.

The composition according to any one of Items 1 and 4, wherein the composition has a particle size of 300 nm or less.

Item 6.

The composition according to any one of Items 1 to 5 for the prevention and/or treatment of an infectious disease, such as a surgical site infection, a prosthetic joint infection, a urinary tract infection, a urinary tract infection, a biliary tract infection, a bloodstream infection, pneumonia, a diabetic foot infection, an intraoral infection, or a skin infection.

Item 7.

The composition according to any one of Items 1 to 6, which is for being carried by a medical material, a medical device, or a biological component.

Item 8.

The composition according to any one of Items 1 to 7, wherein cetylpyridinium chloride hydrate is capable of exhibiting an antimicrobial or bactericidal effect in a sustained manner.

Item 9.

The composition according to any one of Items 1 to 8, which is sterile-filterable.

Item 10.

A medical material or medical device carrying the composition according to any one of Items 1 to 9.

Item 11.

The compound of formula (I) or a salt thereof according to any one of Items 1 to 4, which enables an antimicrobial or bactericidal effect of an antimicrobial agent or bactericidal agent to be exhibited in a sustained manner by mixing the compound of formula (I) or a salt thereof with cetylpyridinium chloride hydrate and carrying the mixture by a medical material, medical device, or biological component.

Item 12.

A method for preventing and/or treating an infectious disease by allowing the composition of any one of Items 1 to 9 to be carried by a medical material or medical device and placing the medical material or medical device in the body, or by spraying or applying the composition onto a biological component.

Item 13.

A method for producing the composition of any one of Items 1 to 9, comprising the following steps:

step 1: forming micelles using an anionic surfactant and a nonionic surfactant;
step 2: adding cetylpyridinium chloride hydrate and a silicon compound represented by formula (I) or a salt thereof to the micelles obtained in step 1;
step 3: removing excess reagent by dialysis to obtain a composition; and
step 4: sterile-filtering the composition obtained in step 3.

Advantageous Effects of Invention

In the present invention, cetylpyridinium chloride hydrate is mixed with a silicon compound to form nanoparticles, thereby enabling efficient enclosure of the drug in the composition. The composition of the present invention can be in the form of nanoparticles having a particle size that allows for sterile filtration. The composition of the present invention can also be carried by a medical material, medical device, or a biological component, to exert the efficacy of cetylpyridinium

chloride hydrate, such as antiviral activity, antibacterial activity, and a bacterial adhesion inhibitory effect or bacterial growth inhibitory effect, in a sustained manner, thereby preventing and/or treating an infectious disease at a surgical field and/or a postoperative affected area.

Brief Description of Drawings

Fig. 1 is a cross-sectional view showing an embodiment of an inactivation filter device.
Fig. 2 is a cross-sectional view showing another embodiment of the inactivation filter device.
Fig. 3 is a schematic diagram showing an example of an air treatment device comprising an inactivation filter device.

Description of Embodiments

The present invention includes the specific embodiments described below as examples, and also includes any combination of the specific embodiments.

In the present specification, the lower alkyl include, for example, a $C_{1-8}$ linear or branched alkyl group ($C_{1-8}$). Specific examples include methyl, ethyl, n-propyl, isopropyl, n-butyl, isobutyl, sec-butyl, tert-butyl, n-pentyl, 1-ethylpropyl, isopentyl, neopentyl, n-hexyl, 1,2,2-trimethylpropyl, 3,3-dimethylbutyl, 2-ethylbutyl, isohexyl, 3-methylpentyl, n-heptyl, isoheptyl, n-octyl, isooctyl, and the like. The lower alkyl is preferably $C_{1-6}$ alkyl.

In the present specification, the lower alkylene include, for example, a $C_{1-8}$ linear or branched divalent hydrocarbon group ($C_{1-8}$ alkylene). Specific examples include methylene, ethylene, n-propylene, isopropylene, n-butylene, isobutylene, n-pentylene, ethylpropylene, isopentylene, n-hexylene, 2-ethylbutylene, isohexylene, 3-methylpentylene, n-heptylene, isoheptylene, n-octylene, isooctylene, and the like. The lower alkylene is preferably $C_{1-6}$ alkylene, and more preferably $C_{1-3}$ alkylene.

In the present specification, the lower alkenyl includes, for example, a $C_{2-6}$ linear or branched alkenyl group ($C_{2-6}$ alkenyl) having one to three double bonds. The lower alkenyl include both a trans form (E form) and a cis form (Z form). Specific examples include vinyl, 1-propenyl, 2-propenyl, 1-methyl-1-propenyl, 2-methyl-1-propenyl, 2-methyl-2-propenyl, 2-butenyl, 1-butenyl, 3-butenyl, 2-pentenyl, 1-pentenyl, 3-pentenyl, 4-pentenyl, 1,3-butadienyl, 1,3-pentadienyl, 2-penten-4-yl, 2-hexenyl, 1-hexenyl, 5-hexenyl, 3-hexenyl, 4-hexenyl, 3,3-dimethyl-1-propenyl, 2-ethyl-1-propenyl, 1,3,5-hexatrienyl, 1,3-hexadienyl, 1,4-hexadienyl, and the like. The lower alkenyl is preferably $C_{2-4}$ alkenyl.

In the present specification, the lower alkanoyl includes, for example, a $C_{1-6}$ linear or branched alkanoyl group ($C_{1-6}$ alkanoyl). Specific examples include formyl, acetyl, propionyl, butyryl, isobutyryl, pentanoyl, tert-butylcarbonyl, hexanoyl, and the like. The lower alkanoyl is preferably $C_{1-4}$ alkanoyl.

In the present specification, the lower alkoxy includes, for example, a $C_{1-8}$ linear or branched alkoxy (lower alkyl-O-) group ($C_{1-8}$ alkoxy). Specific examples include methoxy, ethoxy, n-propoxy, isopropoxy, n-butoxy, isobutoxy, sec-butoxy, tert-butoxy, n-pentyloxy, 1-ethylpropyloxy, isopentyloxy, neopentyloxy, n-hexyloxy, 1,2,2-trimethylpropyloxy, 3,3-dimethylbutyloxy, 2-ethylbutyloxy, isohexyloxy, 3-methylpentyloxy, n-heptyloxy, isoheptyloxy, n-octyloxy, isooctyloxy, and the like. The lower alkoxy is preferably $C_{1-6}$ alkoxy, and more preferably $C_{1-3}$ alkoxy.

In the present specification, the heterocycle includes, for example, a saturated or unsaturated 3- to 12-membered monocyclic or polycyclic heterocycle containing one to five heteroatoms independently selected from the group consisting of nitrogen, oxygen, and sulfur as ring-constituting atom(s). The "unsaturated" ring refers to an aromatic ring or a ring in which the bonds between ring atoms are partially hydrogenated. Specific examples of the heterocycle include a saturated or unsaturated 3-to 12-membered monocyclic or bicyclic heterocycle containing at least one nitrogen as a ring-constituting heteroatom. More specific examples include pyrrole, imidazole, pyrazole, pyridine, tetrahydropyridine, pyrimidine, pyrazine, pyridazine, triazole, tetrazole, dihydrotriazine, azetidine, pyrrolidine, imidazolidine, piperidine, pyrazoline, piperazine, azepane, 1,4-diazepane, indole, indoline (dihydroindoline), isoindole, isoindoline (dihydroisoindoline), benzimidazole, dihydrobenzimidazole, indazole (dihydroindazole), indazoline, quinoline, dihydroquinoline, tetrahydroquinoline, benzotriazole, tetrazolopyridine, tetrazolopyridazine, isoquinoline, dihydroisoquinoline, dihydrotriazine, imidazopyridine, naphthyridine, tetrahydronaphthyridine, hexahydronaphthyridine, cinnoline, quinoxaline, dihydroquinoxaline, tetrahydroquinoxaline, quinazoline, dihydroquinazoline, tetrahydroquinazoline, pyrazolopyridine, hexahydropyrimidopyridine (1,5,7-triazabicyclo[4.4.0]dec-5-ene), and the like. The heterocycle is preferably a saturated or unsaturated 3- to 12-membered bicyclic heterocycle containing at least one nitrogen as a ring-constituting heteroatom.

The composition in the present specification may comprise cetylpyridinium chloride hydrate and a silicon compound. Cetylpyridinium chloride hydrate may be chemically or physically mixed with one or more silicon compounds. For example, cetylpyridinium chloride hydrate may be encapsulated or enclosed in a silicon compound or chemically bonded to a silicon compound to form a nanoparticle composition in the form of a liquid, particles, or a capsule. Cetylpyridinium chloride hydrate can exhibit sustained virus inactivation action and antimicrobial or bactericidal activity

by forming a nanoparticle composition with a silicon compound of formula (I).

The silicon compound in the present specification is a compound represented by formula (I) (hereinafter also referred to as "compound (I)") and includes geometric isomers, stereoisomers, optical isomers, and tautomers. The isomers may be separated and purified by, for example, general optical resolution methods, or may be produced from suitable optically active starting compounds.

The cetylpyridinium chloride hydrate and compound (I) may form acid addition salts or base addition salts depending on the type of their substituents. Examples of acids that form such acid addition salts include inorganic acids, such as hydrochloric acid, hydrobromic acid, nitric acid, sulfuric acid, and phosphoric acid; gluconic acid, methanesulfonic acid, p-toluenesulfonic acid, acetic acid, citric acid, tartaric acid, maleic acid, fumaric acid, malic acid, lactic acid, and the like. Examples of bases that form such base addition salts include inorganic bases, such as sodium hydroxide, potassium hydroxide, calcium hydroxide, sodium carbonate, potassium carbonate, sodium hydrogencarbonate, and potassium hydrogencarbonate; organic bases, such as methylamine, diethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyrrodine, picoline, and choline; and ammonium salts. The salts of the antiviral, and antimicrobial or bactericidal agent and compound (I) can be produced by subjecting them to a common salt-forming treatment.

The composition in the present specification can be used for the prevention and/or treatment of infectious diseases, such as those caused by viruses, bacteria, anaerobic bacteria, or drug-resistant bacteria. Examples of viruses include enveloped viruses including the novel coronavirus (COVID-19) and influenza virus. Examples of bacteria, anaerobic bacteria, or drug-resistant bacteria include Gram-positive bacteria, Gram-negative bacteria, staphylococci, enterococci, *Escherichia coli,* methicillin-nonsusceptible bacteria, vancomycin-nonsusceptible bacteria, penicillin-nonsusceptible bacteria, clarithromycin-nonsusceptible bacteria, and metronidazole-nonsusceptible bacteria. Specific examples include methicillin-resistant *Staphylococcus aureus,* efflux-related methicillin-resistant *Staphylococcus aureus,* vancomycin-intermediate *Staphylococcus aureus,* hetero-vancomycin-intermediate *Staphylococcus aureus,* vancomycin-resistant *Staphylococcus aureus,* vancomycin-resistant enterococci, and the like. Examples of infectious diseases include, but are not limited to, surgical site infections (e.g., surgical-wound infections), prosthetic joint infections, urinary tract infections, biliary atresia, bloodstream infections (e.g., sepsis), pneumonia (e.g., hospital-acquired pneumonia or community-acquired pneumonia), diabetic foot infections, and skin infections such as cellulitis, boils, abscesses, sties, and impetigo.

The composition in the present specification may be carried by chemically or physically attaching or bonding the composition to a medical material or a medical device, or by applying the composition to a biological component at a surgical site, a surgical field, or a postoperative affected area. The composition may also be carried by coating a medical material, a medical device, or an oral biological component with a solution (e.g., aqueous solution) of the composition or spraying a solution (e.g., aqueous solution) of the composition onto a medical material, a medical device, or an oral biological component.

The composition in the present specification can be combined with a pharmaceutically acceptable carrier to prepare a preparation, such as a liquid, an injection, a spray, an aerosol, a lotion, an ointment, a cream, a gel, a patch, a tape, or a cataplasm. Examples of pharmaceutically acceptable carriers include activators, such as lactose, sucrose, sodium chloride, glucose, urea, starch, calcium carbonate, kaolin, and crystalline cellulose; binders, such as water, ethanol, propanol, simple syrup, a glucose solution, a starch solution, a gelatin solution, carboxymethylcellulose, shellac, methylcellulose, potassium phosphate, and polyvinylpyrrolidone; lubricants, such as purified talc, stearic acid salts, powdered boric acid, and polyethylene glycol; and the like.

In the present specification, examples of medical materials include titanium metal, ceramics, functional resins, functional plastics, polyurethane, polyvinyl chloride, cotton, silk, hemp, nonwoven fabrics, and the like.

In the present specification, the medical device is a prosthetic medical device that can be implanted and/or placed in the body, and is produced from a medical material. Examples of medical devices include catheters, pacemakers, grafts, stents, wires, guidewires, orthopedic implants, medical implants, bodily implants, implantable diffusion pumps, injection ports, heart valves, prosthetic joints, aortic grafts, blood oxygenation membranes, blood oxygenation tubing, dialysis membranes, drug delivery systems, endoprostheses, endotracheal tubes, intra-aortic balloons, vascular grafts, vascular tubing, arterial tubing, and venous tubing.

In the present specification, examples of biological components include skin, bones (e.g., joints), organs (e.g., urinary tract, biliary tract, and lung), and the like. The biological component is preferably a biological component at a surgical site, a surgical field, or a postoperative affected area.

In the present specification, sterile filtration refers to filtration using a filter with a pore size (e.g., 0.2 to 0.45 $\mu$m) through which bacteria etc. cannot pass, preferably a filter with a pore size of 0.22 $\mu$m, and is preferably ultrafiltration. The composition in the present specification may have a particle size that allows for sterile filtration. The average particle size of the composition can be measured, for example, by dynamic light scattering. The composition has an average particle size of, for example, 300 nm or less, preferably 10 to 250 nm, and more preferably 50 to 230 nm.

It is also a preferred embodiment that the nanoparticles of the present specification are attached to a filter and

then used as a filter device mounted in an air cleaning device.

In this case, the method for attaching the nanoparticles in the present specification to the filter can be selected from a wide range of known methods and is not particularly limited. Specific examples include a method in which the nanoparticles in the present specification are added to water to prepare a colloidal liquid or suspension, the colloidal liquid or suspension is sprayed onto a filter or a filter is dipped into the colloidal liquid or suspension, and then the filter is dried to adhere the composition to the filter.

An example of an embodiment of the above filter device is described below in detail.

The filter member may have a single layer structure or a multilayer structure composed of several layers. Examples of usable materials of the filter member include, but are not particularly limited to, cotton, blended fabrics, paper, gauze, nonwoven fabrics, lint, and chemical fibers, such as acrylic fibers, polyester fibers, and urethane foam. To keep these materials in the layer shape, forming by suitable compression can be utilized, or a wire mesh or resin runner, which is a component that maintains the layer structure of the filter, can be used.

The nanoparticles of the present invention can be applied in the first type in which they are placed on one surface of a layer of a filter member or between layers of a filter member, or the second type in which they are attached to the fibers of a filter member or placed in voids between the fibers. Moreover, the first and second types can be used in combination. As a method for attaching or adding the composition of the present invention to the filter member, for example, a method of immersion in an aqueous solution of the nanoparticles of the present specification or spraying of the aqueous solution can be used.

The filter device preferably has a moisture-permeable waterproof layer on each surface of the layer of the filter member, i.e., the intake side for introducing air to be treated and the discharge side for air to be treated.

The moisture-permeable waterproof layer is a layer that simultaneously exhibits waterproof and dustproof properties and air permeability, and has both water resistance and air permeability as a basis. Water resistance is a numerical representation of how much water pressure the surface of a layer can withstand without the leakage of water to the back side. The air permeability is expressed by the time required for 100 cc of air to pass through under a pressure of 1.27 kPa per 6.45 $cm^2$ area (Gurley number, sec, described in JISP8117). The air permeability corresponds to the inverse of the flow rate, and the smaller the Gurley number, the higher the permeability. As the characteristics of moisture permeability and waterproofness, the Gurley number is generally 0.008 to 30, and preferably 0.01 to 10, and the water resistance is generally 3 to 200 kPa, and preferably 5 to 100 kPa. The moisture-permeable waterproof layer can be composed of, for example, a porous membrane of a PTFE (polytetrafluoroethylene) resin.

The moisture-permeable waterproof layer, which has both water resistance and air permeability, functions to retain moisture in the layer portion of the filter member while allowing air to be treated to pass through, on both the intake side and the discharge side of the layer of the filter member including the nanoparticles of the present specification. Thus, humidity or moisture can be retained in the layer of the filter member including the nanoparticles of the present specification to maintain moisture in the layer, despite the presence of a flow of air to be treated.

An example of an embodiment of the filter device is described with reference to drawings.

Fig. 1 shows an example in which a layer of the nanoparticles of the present specification is provided separately from a filter member. An inactivation filter device 10 is a filter device for capturing and inactivating viruses, such as coronaviruses and/or bacteria, such as Mycobacterium tuberculosis. The filter device 10 includes a layer 20 of a filter member as a capture or collection element. For example, coronaviruses are as small as 0.1 μm in diameter, but the layer 20 of the filter member can be composed of a member that has a collection rate of 99.97% or more for particles with a particle size of 0.3 μm, like a HEPA filter. This is because small coronaviruses have multiple opportunities to come into contact with the filter member, and droplets containing coronaviruses are a few micrometers in size.

One side 20i of the layer 20 of the filter member is an intake side for introducing air to be treated, and the other side 20o of the layer 20 is a discharge side for air to be treated. Here, a layer 30 of the nanoparticles of the present specification is provided on the one side 20i of the layer 20, which is the air discharge side. The layer 30 of the nanoparticles of the present specification can be formed by directly spraying the nanoparticles of the present specification onto the one side 20i of the layer 20 of the filter member or by attaching a fiber layer impregnated with the nanoparticles of the present specification to the one side 20i of the layer 20.

The example mentioned above only includes the layer 20 of the filter member and the layer 30 of the nanoparticles of the present specification, whereas the inactivation filter device 10 includes individual moisture-permeable waterproof layers 40, 50 on the intake side and the discharge side. The moisture-permeable waterproof layers 40, 50 are layers that have both waterproofness and air permeability and can be composed of, for example, a porous membrane of a PTFE (polytetrafluoroethylene) resin.

In the inactivation filter device 10, sufficient moisture is contained in the layer 20 of the filter member and the layer 30 of the nanoparticles of the present specification in order for the inactivation action by the layer 30 of the nanoparticles of the present specification to be effectively exhibited. However, such moisture is susceptible to loss by air flowing through the inactivation filter device 10. In this regard, the inactivation filter device 10 has the moisture-permeable waterproof layers 40, 50 individually on the intake side and the discharge side, thereby effectively preventing loss of moisture in the layer 20

of the filter member and the layer 30 of the nanoparticles of the present specification.

Fig. 2 shows another embodiment in which the nanoparticles of the present specification are provided inside a filter member. An inactivation filter device 100 according to another embodiment is in the form in which the inactivating agent is retained in a layer 200 of a filter member for capturing viruses and/or bacteria. The inactivating agent is attached to the fibers in the layer 200 of the filter member or retained in voids between the fibers. The inactivating agent in the layer 200 is adjacent to viruses and the like captured by the layer 200, which makes it easier to come in contact with the viruses and the like, thereby enabling more effective inactivation.

The inactivation filter device 100 also has individual moisture-permeable waterproof layers 40, 50 on the intake side and discharge side of the layer 200 of the filter member. This makes it possible to effectively prevent loss of moisture in the filter member 200 including the inactivating agent without inhibiting the flow of air to be treated.

The inactivation filter devices 10, 100 can generally be used in combination with a deodorizing filter or a dust collection filter at the air inlet of an air cleaner or the like.

Fig. 3 is a schematic diagram showing another example of the use of the inactivation filter devices 10, 100. A tank 60 include the inactivation filter device 10 (100) inside and has a structure in which air to be treated is taken in from the bottom of the tank, and the treated air is discharged from the top of the tank.

Inside the tank 60, a moisture-permeable waterproof sheet 62 is disposed in the lower portion. The moisture-permeable waterproof sheet 62 prevents water leakage while allowing air flow. Thus, by providing an aqueous layer 64 on the moisture-permeable waterproof sheet 62, air to be treated that is introduced from the bottom can be bubbled and allowed to flow toward the upper portion of the tank 60 in a highly humid state. In the upper portion of the tank 60, a visualization means 66 for visualizing viruses and an inactivation filter device 10 (100) are disposed. The visualization means 66 located on the front can detect whether air to be treated contains coronaviruses by visualizing viruses. Examples of the visualization means 66 include an ostrich antibody filter, a fluorescent protein-carrying filter that carries a fusion protein comprising a VHH antibody that binds to a virus and a highly bright fluorescent protein, and the like. After air introduced into the tank 60 is bubbled and allowed to flow for a certain period of time, the visualization means 66 can be taken out to detect the presence of viruses. In this case, when the visualization means 66 is an ostrich antibody filter, the ostrich antibody filter taken out is sprayed with a secondary antibody solution in the dark and then irradiated with LED light or black light. When it glows yellowish green by the irradiation, the presence of coronaviruses can be detected. When the visualization means 66 is a fluorescent protein-carrying filter, the presence of coronaviruses cab be detected by observation with a fluorescence microscope.

The inactivation filter device 10 (100) located on the back side of the visualization means 66 effectively captures and inactivates viruses and the like in the air passing through it, and allows safe air to escape from the discharge port to the outside.

The moisture-permeable waterproof layer 40 on the intake side and the moisture-permeable waterproof layer 50 on the discharge side generally have similar characteristics. However, for example, when highly humid air is introduced into the inactivation filter device 10 (100) by bubbling as shown in the above example of use, the moisture-permeable waterproof layer 40 on the intake side can be made more permeable to humidity than the moisture-permeable waterproof layer 50 on the discharge side. This makes it possible to efficiently introduce highly humid air to be treated into the inactivation filter device 10 (100).

Typical Method for Producing Silicon Compound (I)

The silicon compound represented by formula (I) may be a commercially available product or may be newly produced according to a usual method in the art, for example, the production methods described below. The method for producing the compound represented by formula (I) is not limited to the production methods described below. In the following production methods, each starting material compound may form a salt if it does not inhibit reactions. The salt for use can be those listed as examples of salts of compound (I).

For starting material compounds whose specific production method is not described, commercially available products may be used, or the starting material compounds may be produced by known methods or similar methods. In each reaction in the following production methods, the product in the form of a reaction solution or of a crude product may be used in the subsequent reaction, isolation from the reaction mixture can be performed according to a usual method, or purification can be easily performed by an ordinary separation technique. Examples of ordinary separation techniques include extraction, concentration, crystallization, filtration, recrystallization, distillation, and various chromatographies. The products obtained in the following production methods may be each isolated and purified as a free compound, or a salt or solvate thereof.

In the following production methods, when an alkylation reaction, a hydrolysis reaction, an amination reaction, an esterification reaction, an amidation reaction, a urea derivatization reaction, an etherification reaction, an oxidation reaction, a reduction reaction, or the like is performed, these reactions can be performed according to known methods or similar methods. These methods are, for example, those described in Organic Functional Group Preparation (2nd edition,

Academic Press, Inc., issued in 1989), Comprehensive Organic Transformations (VCH Publishers Inc., issued in 1989), and Greene's Protective Groups in Organic Synthesis (authored by P. G. M. Wuts and T. W. Greene, 4th edition, 2006).

Typical Method 1 for Producing Silicon Compound (I) (Amidation Reaction)

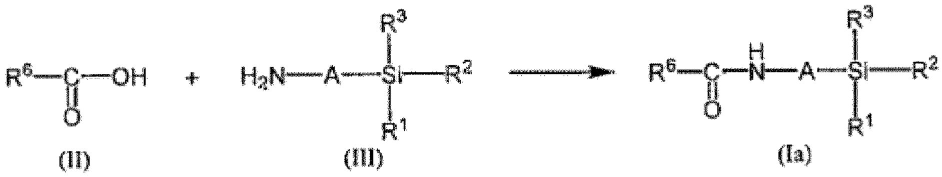

wherein each symbol is as defined in the present specification.

Compound (Ia) can be obtained by amidation of compound (II) and compound (III). The amidation can be performed by a method commonly used by those skilled in the art. Compound (Ia) may be produced by reacting compound (II) or a reactive derivative thereof at the carboxy group and compound (III) or a reactive derivative thereof at the amino group.

Examples of reactive derivatives at the carboxy group of compound (II) include acid halides, acid azides, acid anhydrides, activated amides, activated esters, and the like. Preferred examples of reactive derivatives include an acid chloride, an acid azide; a mixed acid anhydride with an acid, such as substituted phosphoric acid (e.g., dialkylphosphoric acid, phenylphosphoric acid, diphenylphosphoric acid, dibenzylphosphoric acid, or halogenated phosphoric acid), dialkylphosphorous acid, sulfurous acid, thiosulfuric acid, sulfuric acid, sulfonic acid (e.g., methanesulfonic acid), an aliphatic carboxylic acid (e.g., acetic acid, propionic acid, butyric acid, isobutyric acid, pivalic acid, pentanoic acid, isopentanoic acid, 2-ethylbutyric acid, or trichloroacetic acid), or an aromatic carboxylic acid (e.g., benzoic acid); a symmetrical acid anhydride; an activated amide with imidazole, 4-substituted imidazole, dimethylpyrazole, triazole, or tetrazole; an activated ester (e.g., cyanomethyl ester, methoxymethyl ester, dimethyliminomethyl ester, vinyl ester, propargyl ester, p-nitrophenyl ester, 2,4-dinitrophenyl ester, trichlorophenyl ester, pentachlorophenyl ester, or mesylphenyl ester); an ester with an N-hydroxy compound (e.g., N,N-dimethylhydroxylamine, 1-hydroxy-2-(1H)-pyridone, N-hydroxysuccinimide, N- hydroxyphthalimide, or HOBt); and the like. Any reactive derivative can be selected from among these depending on the chemical properties of compound (II) to be used.

In the above reaction, when compound (II) is used in a free acid form or its salt form, the reaction is preferably performed in the presence of a condensing agent. Examples of condensing agents include a wide variety of condensing agents known in the art, such as DCC; N-cyclohexyl-N'-morpholinoethyl carbodiimide; N-cyclohexyl-N'-(4-diethylaminocyclohexyl) carbodiimide; diethyl carbodiimide; N,N'-diethyl carbodiimide; N,N'-diisopropyl carbodiimide; WSC or its HCl salt; N,N'-carbonylbis(2-methylimidazole); pentamethylene ketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenylphosphoryl azide; thionyl chloride; oxalyl chloride; lower alkyl haloformates, such as ethyl chloroformate and isopropyl chloroformate; triphenylphosphine; 2-ethyl-7-hydroxybenzisoxazolium salts; 2-ethyl-5-(m-sulfophenyl) isoxazolium hydroxide inner salts; benzotriazol-1-yloxy-tris(dimethylamino)phosphonium hexafluorophosphate; 1-(p-chlorobenzenesulfonyloxy)-6-chloro-1H-benzotriazole; Vilsmeier reagents prepared by reacting DMF with thionyl chloride, phosgene, trichloromethyl chloroformate, phosphorus oxychloride, etc.; and the like. It is more preferable to perform the reaction in the presence of a condensing agent described above together with an activated ester such as N-hydroxysuccinimide, N-hydroxyphthalimide, or HOBt.

Preferred examples of reactive derivatives at the amino group of compound (III) include a Schiff base-type imino or an enamine-type tautomer thereof formed by a reaction of compound (III) with a carbonyl compound, such as an aldehyde or a ketone; a silyl derivative formed by a reaction of compound (III) with a silyl compound, such as bis(trimethylsilyl)acetamide, mono(trimethylsilyl)acetamide, or bis(trimethylsilyl)urea; a reactive derivative formed by a reaction of compound (III) with phosphorus trichloride, phosgene, or the like; and the like.

This reaction can generally be performed in a common solvent that does not adversely affect the reaction. Examples of solvents include water; alcohol solvents, such as MeOH, EtOH, isopropanol, n-butanol, trifluoroethanol, and ethylene glycol; ketone solvents, such as acetone and methyl ethyl ketone; ether solvents, such as THF, dioxane, Et2O, diisopropyl ether, and diglyme; ester solvents, such as AcOMt and AcOEt; aprotic solvents, such as MeCN, DMF, and DMSO; hydrocarbon solvents, such as n-pentane, n-hexane, n-heptane, and cyclohexane; halogenated hydrocarbon solvents, such as methylene chloride, ethylene chloride, and DCM; other organic solvents; mixed solvents of these; and the like.

This reaction may be performed in the presence of a base. Examples of bases include a wide variety of known

inorganic bases and organic bases. Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., Li2CO3, Na2CO3, K2CO3, and Cs2CO3), alkali metal lower alkoxides (e.g., sodium methoxide and sodium ethoxide), and alkali metal hydrides (e.g., NaH and KH). Examples of organic bases include trialkyl amines (e.g., trimethylamine, triethylamine, and N-ethyldiisopropylamine), pyridine, quinoline, piperidine, imidazole, picoline, dimethylaminopyridine, dimethylaniline, N-methylmorpholine, DBN, DABCO, DBU, and the like. If these bases are liquid, they can also be used as a solvent. These bases may be used singly or in a combination of two or more. The amount of the base for use is generally 0.1 to 10 moles, and preferably 0.1 to 3 moles, per mole of compound (II).

The proportion of compound (II) and compound (III) in typical production method 1 is such that the amount of the former is generally at least 1 mole, and preferably 1 to 5 moles, per mole of the latter. The reaction temperature is not particularly limited, and the reaction is generally performed under cooling, room-temperature, or heating conditions. The reaction is performed at a temperature ranging from preferably room temperature to 100°C for, for example, 30 minutes to 30 hours, preferably 30 minutes to 5 hours. When compound (1a) has an **N-**protecting group, the N-protecting group can be removed by a common method, such as hydrolysis or hydrogenolysis.

Typical Method 2 for Producing Silicon Compound (I) (Urea Derivatization Reaction)

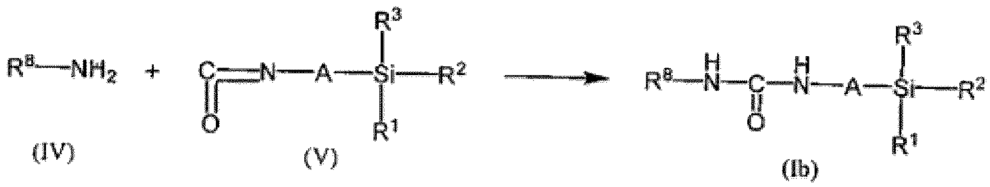

wherein each symbol is as defined in the present specification.

Compound (Ib) can be obtained by urea derivatization of compound (IV) and compound (V). The urea derivatization may be urea derivatization commonly used by those skilled in the art. This reaction can be generally performed in an inert solvent that does not adversely affect the reaction. Examples of inert solvents include the ketone solvents, ether solvents, alcohol solvents, ester solvents, aprotic solvents, and halogenated hydrocarbon solvents described in typical production method 1; other organic solvents; mixed solvents of these; water; and the like.

This reaction may be performed in the presence of a base, if necessary. Examples of bases include the bases described in typical production method 1. These bases may be used singly or in a combination of two or more. The amount of the base for use is generally 0,1 to 10 moles, and preferably 0.1 to 3 moles, per mole of compound (IV).

The proportion of compound (IV) and compound (V) in typical production method 2 is such that the amount of the former is generally about at least 1 mole, and preferably about 1 to 5 moles, per mole of the latter.

The reaction temperature is not particularly limited, and the reaction is generally performed under cooling, room-temperature, or heating conditions. The reaction is performed at a temperature ranging from preferably room temperature to 100°C for, for example, 30 minutes to 30 hours, preferably 30 minutes to 5 hours.

Typical Method for Producing Drug-containing Silicon Nanoparticle Composition

The composition of the present invention can be produced, for example, by mixing cetylpyridinium chloride hydrate and a silicon compound (I) according to the method described in NPL 1. Specifically, the method for producing the composition of the present invention comprises the following steps.

Step 1: Formation of micelle

An anionic surfactant, such as Aerosol OT (AOT; dioctyl sodium sulfosuccinate), a nonionic surfactant, and 1-butanol, ethylene glycol, 1,3-propanediol, 1,4-butanediol, 1,6-hexanediol, or like diol are dissolved in water, followed by mixing with vigorous stirring, thereby forming micelles.

Step 2: Formation of Drug-micelle

A drug (i.e., an antimicrobial agent or a bactericidal agent) dissolved in N,N-dimethylformamide (DMF) is added to the micelles obtained in step 1, and they are mixed to form drug-micelles.

Step 3: Formation of drug-silica-micelle

A silicon compound is added to the drug-micelles obtained in step 2, and the mixture is stirred to form drug-silica-micelles. In this step, as the silicon compound, one or more different silicon compounds may be added simultaneously or separately.

Step 4: Dialysis

After the drug-silica-micelles are formed in step 3, the surfactants, solvent, and excess reagent are removed by dialysis to obtain drug-silica nanoparticles.

Step 5: Sterile formulation

The drug-silica nanoparticles obtained in step 4 are filtered through a sterile filter to obtain a sterilized composition.
The composition obtained above is also preferably solidified (made solid) by performing freeze-drying or the like to remove the solvent.
The composition of the present invention obtained as described above can be carried by a medical material or a medical device, or a biological component at a surgical field or a postoperative affected area, by coating, specifically by an immersion method, a spraying method, or the like.
The present invention is described in more detail below with reference to Reference Examples, Production Examples, Examples, and Experimental Examples; however the present invention is not limited thereto.
In the present specification, the following abbreviations may be used.

STR: structural formula
DBN: 1,5-diazabicyclo[4.3.0]non-5-ene
DABCO: 1,4-diazabicyclo[2.2.2]octane
DBU: 1,8-diazabicyclo[5.4.0]-7-undecene
HOBt: 1-hydroxybenzotriazole
DCC: dicyclohexylcarbodiimide
WSC: 3-ethyl-1-(3-methylaminopropyl)carbodiimide
AcOMt: methyl acetate
AcOEt: ethyl acetate
MeCN: acetonitrile
MeOH: methanol
EtOH: ethanol
THF: tetrahydrofuran
DCM: dichloromethane
DMF: N,N-dimethylformamide
DMSO: dimethyl sulfoxide
$Et_2O$: diethyl ether
Hexane: hexane
LiOH: lithium hydroxide
NaOH: sodium hydroxide
KOH: potassium hydroxide
NaH: sodium hydride
KH: potassium hydride
$Li_2CO_3$: lithium carbonate
$Na_2CO_3$: sodium carbonate
$K_2CO_3$: potassium carbonate
$Cs_2CO_3$: cesium carbonate
AOT: dioctyl sodium sulfosuccinate
Z-Asp(OBzl)-OH: (S)-4-(benzyloxy)-2-benzyloxycarbonylamino)-4-oxobutanoic acid
Z-Asp-obzl: (S)-4-(benzyloxy)-2-benzyloxycarbonylamino)-4-oxobutanoic acid
Z-His(Bzl)-OH: (S)-3-(1-benzyl-1H-imidazol-4-yl)-2-(benzyloxycarbonylamino)propionic acid
Pd-C: palladium on carbon
TLC: thin-layer chromatography

In the following Examples, "room temperature" generally refers to about 10 to 35°C. A ratio indicated for a mixed solvent refers to a volume ratio, unless otherwise specified. Unless otherwise specified, % refers to mass%.

Proton nuclear magnetic resonance ([1]HNR) spectra were measured by Fourier transform NMR (using any of Bruker AVANCE 300 (300 MHz), Bruker AVANCE III 400 (400 MHz), and Bruker AVANCE III 500 (500 MHz)) at room temperature.

Reference Examples

Production of Intermediate for Silicon Compound

Intermediate for Silicon Compound

Table 1 shows the structures and names of synthesized compounds.

Reference Example 1: Synthesis of Compound (1)

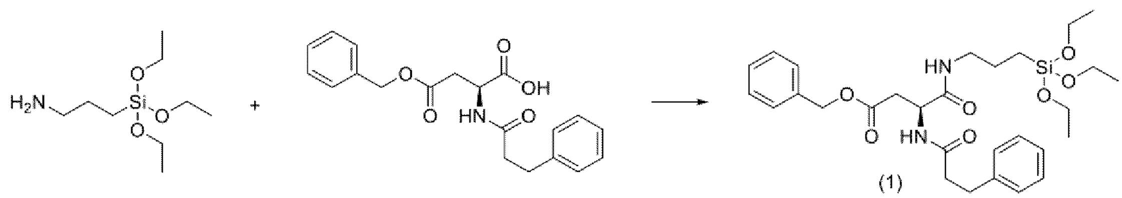

(1)

Z-Asp(OBzl)-OH (836 mg) and HOBt (16.3 mg) were dissolved in dichloromethane (10 ml), and 1-(3-dimethylaminopropyl)-3-ethyl-carbodiimide (0.41 ml) was added, followed by stirring for a while. Thereafter, 3-aminopropyltriethoxysilane (0.5 ml) was added, and the mixture was stirred at room temperature. The reaction mixture was purified using a florisil column (eluent: hexane:CH2Cl2 = 1:1-0:1) to obtain compound (1) (837 mg) as a colorless solid.
$^1$H NMR (CDCl3, 400 MHz) δ 0.60 (m, 2 H), 1.22 (t, J = 7.0 Hz, 9 H), 1.58 (m, 2 H), 2.72 (dd, J = 17.2 Hz, 6.4 Hz, 1 H), 3.14 (dd, J = 17.2 Hz, 4.2 Hz, 1 H), 3.22 (m, 2 H), 3.81 (q, J = 7.0 Hz, 6 H), 4.55 (m, 1 H), 5.06-5.17 (m, 4 H), 5.91 (d, J = 7.7 Hz, 1 H), 6.53 (m, 1 H), 7.28-7.45 (m, 10 H).

Reference Example 2: Synthesis of Compound (2)

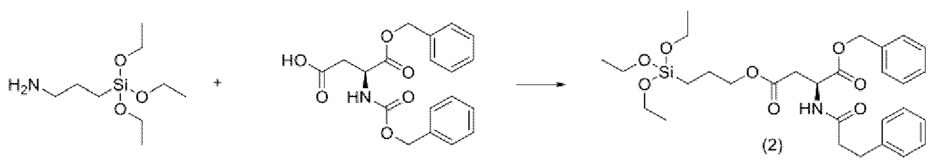

(2)

Z-Asp-obzl (836 mg) and HOBt (16.3 mg) were dissolved in dichloromethane (10 ml), and 1-(3-dimethylaminopropyl)-3-ethyl-carbodiimide (0.41 ml) was added, followed by stirring for a while. Thereafter, 3-aminopropyltriethoxysilane (0.5 ml) was added, and the mixture was stirred at room temperature. The reaction mixture was purified using a florisil column (eluent: hexane:$CH_2Cl_2$ = 1:1-0:1) to obtain compound (2) (649 mg) as a colorless solid.
$^1$H NMR (CDCl3, 400 MHz) δ 0.60 (m, 2 H), 1.21 (t, J = 7.0 Hz, 9 H), 1.58 (m, 2 H), 2.69 (dd, J = 5.7 Hz, 4.1 Hz, 1 H), 2.91 (dd, J =5.7 Hz, 4.1 Hz, 1 H), 3.19 (m, 2 H), 3.81 (q, J = 7.0 Hz, 6 H), 4.61 (m, 1 H), 5.10 (s, 2H), 5.18 (dd, J =16.0 Hz, 12.3 Hz, 2 H), 5.78 (bs, 1 H), 6.08 (d, J = 8.8 Hz, 1 H), 7.28-7.38 (m, 10 H).

Reference Example 3: Synthesis of Compound (3)

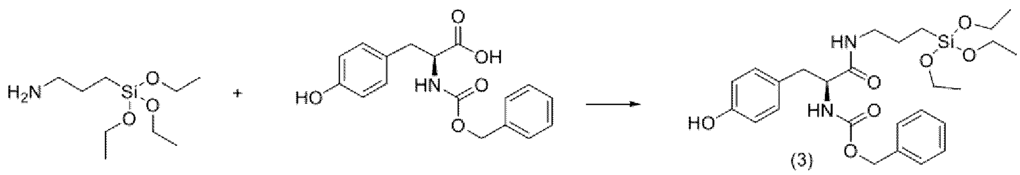

(3)

3-Aminopropyltriethoxysilane (281 mg), N-CBZ-L-tyrosine (400 mg), and HOBt (9.7 mg) were dissolved in dichloromethane (10 ml), and 1-(3-dimethylaminopropyl)-3-ethyl-carbodiimide (197 mg) was added, followed by stirring at room temperature for 1 hour. The reaction mixture was purified using a florisil column (eluent: CH2Cl2:EtOH = 20:1) to obtain compound (3) (31 mg) as a colorless solid.
$^1$H NMR (CDCl3, 400 MHz) δ 0.60-0.68 (m, 2 H), 1.22 (t, J = 7.0 Hz, 9 H), 1.45-1.50 (m, 2 H), 2.85-3.25 (m, 4 H), 3.80 (q, J = 7.0 Hz, 6 H), 4.21-4.33 (m, 1 H), 5.09 (d, J = 3.0 Hz, 2 H), 5.40 (br-s, 1 H), 5.79 (br-s, 1 H), 5.85 (br-s, 1 H), 6.74 (d, J = 8.5 Hz, 2 H), 7.03(br-d, 2 H ), 7.29-7.36 (m, 5 H).

Reference Example 4: Synthesis of Compound (4)

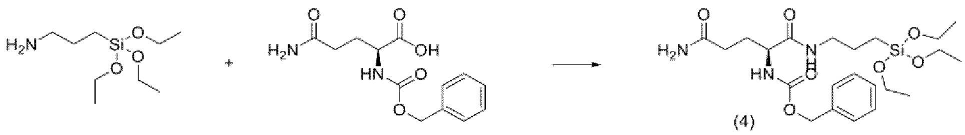

(4)

Aminopropyltriethoxysilane (942 mg), N-carbobenzyloxy-L-glutamine (1193 mg), and HOBt (32.6 mg) were dissolved in dichloromethane (10 ml), and 1-(3-dimethylaminopropyl)-3-ethylcarbodiimide (727 mg) was added, followed by stirring at room temperature for 1 hour. The reaction mixture was purified using a florisil column (eluent: CH2Cl2:EtOH = 20:1) to obtain compound (4) (700 mg) as a white solid.
$^1$H NMR (DMSO-d6, 400 MHz) δ 0.60-0.68 (m, 2 H), 1.14 (t, J = 7.0 Hz, 9 H), 1.39-1.48 (m, 2 H), 1.62-1.75 (m, 1 H), 1.79-1.89 (m, 1 H), 2.00-2.15 (m, 3 H), 2.94-3.07 (m, 3 H), 3.73 (q, J = 7.0 Hz, 6 H), 3.86-3.95 (m, 1 H), 6.75 (s, 1 H), 7. 25 (s, 1 H), 7.26-7.45 (m, 5 H), 7.85 (t, J = 5.4 Hz, 1 H).

Reference Example 5: Synthesis of Compound (5)

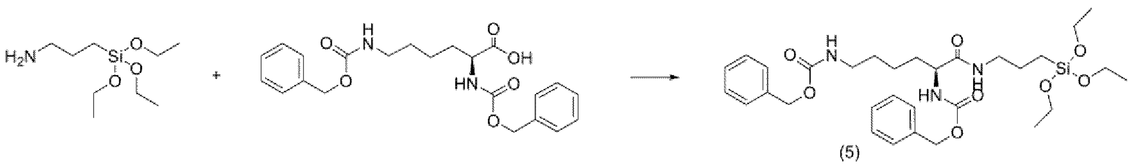

(5)

Aminopropyltriethoxysilane (534 mg), N,N'-dicarbobenzyloxy-L-lysine (1000 mg), and HOBt (18.5 mg) were dissolved in dichloromethane (10 ml), and 1-(3-dimethylaminopropyl)-3-ethyl-carbodiimide (412 mg) was added, followed by stirring at room temperature for 1 hour. The reaction mixture was purified using a florisil column (eluent: CH2Cl2:EtOH = 20:1) to obtain compound (5) (570 mg) as a white solid.
$^1$H NMR (CDCl3, 400 MHz) δ 0.62 (br-t, J = 7.8 Hz, 2 H), 1.22 (t, J = 7.0 Hz, 9 H), 1.32-1.42 (m, 2 H), 1.47-1.56 (m, 2 H), 1.58-1.69 (m, 2 H), 1.80-1.92 (m, 1 H), 3.10-3.30 (m, 4 H), 3.81 (q, J = 7.0 Hz, 6 H), 4.04-4.13 (m, 1 H), 4.85 (br-s, 1 H), 5.01-5.17 (m, 4 H), 5.48 (br-d, J = 6.4 Hz, 1 H), 6.23 (br-s, 1 H), 7.27-7.40 (m, 10 H).

Reference Example 6: Synthesis of Compound (6)

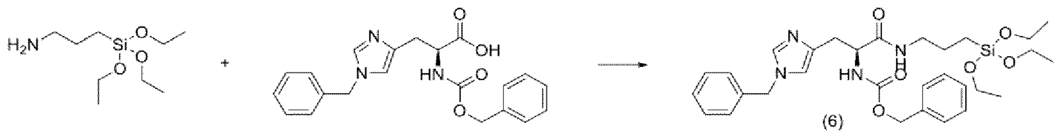

(6)

Aminopropyltriethoxysilane (942 mg), Z-His(Bzl)-OH (1695 mg), and HOBt (33 mg) were dissolved in dichloromethane (10 ml), and 1-(3-dimethylaminopropyl)-3-ethyl-carbodiimide (727 mg) was added, followed by stirring at room temperature for 1 hour. The reaction mixture was purified using a florisil column (eluent: $CH_2Cl_2$) to obtain compound (6) (1520 mg) as a white solid. $^1H$ NMR (CDCl3, 400 MHz) δ 0.50 (br-t, J = 6.1 Hz, 2 H), 1.21 (t, J = 7.0 Hz, 9 H), 1.45-1.55 (m, 2 H), 2.86-2.94 (m, 1 H), 3.09-3.19 (m, 3 H), 3.80 (q, J = 7.0 Hz, 6 H), 4.44 (br-s, 1 H), 5.02 (s, 2H), 5.11 (d, J = 2.0 Hz, 2 H), 6.71 (br-s, 2 H), 6.93 (br-s, 1 H ), 7.10-7.15 (m, 2 H), 7.28-7.42 (m, 10 H).

Reference Example 7: Synthesis of Compound (7)

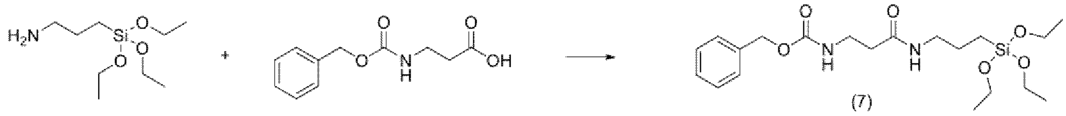

(7)

Aminopropyltriethoxysilane (942 mg), Z-His(Bzl)-OH (1045 mg), and HOBt (33 mg) were dissolved in dichloromethane (10 ml), and 1-(3-dimethylaminopropyl)-3-ethyl-carbodiimide (727 mg) was added, followed by stirring at room temperature for 1 hour. The reaction mixture was purified using a florisil column (eluent: $CH_2Cl_2$) to obtain compound (7) (1100 mg) as a white solid. 1H NMR (CDCl3, 400 MHz) δ 0.63 (br-t, J = 7.9 Hz, 2 H), 1.22 (t, J = 7.0 Hz, 9 H), 1.58-1.67 (m, 2 H), 2.39 (t, J = 5.8 Hz, 2 H), 3.24 (dd, J = 12.8 Hz, 6.8 Hz, 2 H), 3.48 (dd, J = 12.0 Hz, 6.1 Hz, 2 H), 3.81 (q, J = 7.0 Hz, 6 H), 5.09 (s, 2H), 5.48 (br-s, 1 H), 5.85 (br-s, 1 H), 7.29-7.40 (m, 5 H).

Reference Example 8: Synthesis of Compound (8)

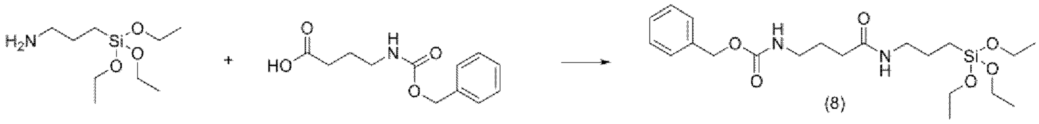

(8)

Aminopropyltriethoxysilane (942 mg), 4-benzyloxycarbonylamino-butyric acid (1111 mg), and HOBt (33 mg) were dissolved in dichloromethane (10 ml), and 1-(3-dimethylaminopropyl)-3-ethyl-carbodiimide (727 mg) was added, followed by stirring at room temperature for 1 hour. The reaction mixture was purified using a florisil column (eluent: $CH_2Cl_2$) to obtain compound (8) (1600 mg) as a white solid.
$^1H$ NMR (CDCl3, 400 MHz) δ 0.64 (br-t, J = 8.2 Hz, 2 H), 1.22 (t, J = 7.0 Hz, 9 H), 1.57-1.73 (m, 5 H), 1.80-1.88 (m, 2 H), 2.17-2.23 (m, 4 H), 2.36 (t, J = 6.4 Hz, 1 H), 3.13-3.28 (m, 6 H), 3.81 (q, J = 7.0 Hz, 6 H), 5.09 (s, 2H), 5.14 (br-s, 1 H), 6.09 (br-s, 1 H), 7.30-7.42 (m, 5 H).

Reference Example 9: Synthesis of Compound (9)

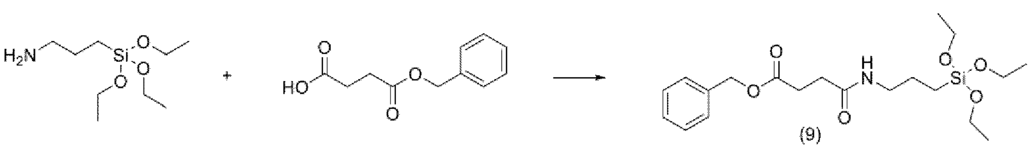

(9)

Aminopropyltriethoxysilane (471 mg), 4-(benzyloxy)-4-oxobutanoic acid (487 mg), and HOBt (16 mg) were dissolved in dichloromethane (10 ml), and 1-(3-dimethylaminopropyl)-3-ethylcarbodiimide (363 mg) was added, followed by stirring at room temperature for 3 hours. The reaction mixture was purified using a florisil column (eluent: $CH_2Cl_2$) to

obtain compound (9) (590 mg) as a colorless oil.

$^1H$ NMR ($CDCl_3$, 400 MHz) δ 0.63 (t, J = 8.0 Hz, 2 H), 1.23 (t, J = 7.0 Hz, 9 H), 1.57-1.67 (m, 2 H), 2.47 (t, J = 6.9 Hz, 2 H), 2.73 (t, J = 6.9 Hz, 2 H), 3.24 (q, J = 6.9 Hz, 2 H), 3.82 (q, J = 7.0 Hz, 6 H), 5.13 (s, 2H), 5.87 (br-s, 1 H), 7.29-7.43 (m, 5 H).

Table 1

| Table 1 | | |
|---|---|---|
| Compound | STR | Name of compound* |
| 1 | 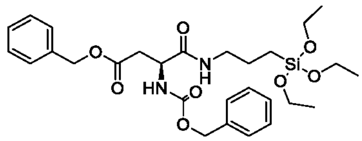 | (S)-benzyl 3-(benzyloxycarbonylamino)-4-oxo-4-(3-(triethoxysilyl)propylamino)butanoate |
| 2 | 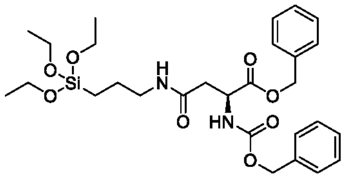 | (R)-benzyl 3-(benzyloxycarbonylamino)-4-oxo-4-(3(triethoxysilyl)propylamino)butanoate |
| 3 | 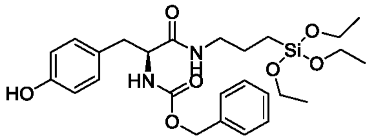 | (S)-benzyl 3-(4-hydroxyphenyl)-1-oxo-1-(3-(triethoxysilyl)propylamino)propan-2-yl carbamate |
| 4 | 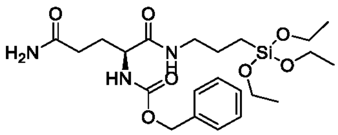 | (S)-benzyl 5-amino-1,5-dioxo-1-(3-(triethoxysilyl)propylamino)pentan-2-yl carbamate |
| 5 | 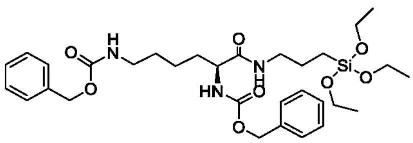 | (S)-benzyl 6-oxo 6-(3-(triethoxysilyl)propylamino)hexane 1,5-diyldicarbamate |
| 6 | 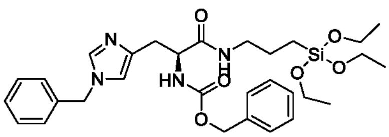 | (S)-benzyl 3-(1-benzyl-1H-imidazol-4-yl)-1-oxo-1-(3-(triethoxysilyl)propylamino)propan-2-yl carbamate |
| 7 | 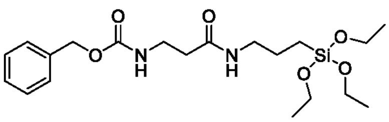 | (S)-benzyl 3-oxo-3-(3-(triethoxysilyl)propylamino)propylcarbamate |
| 8 | 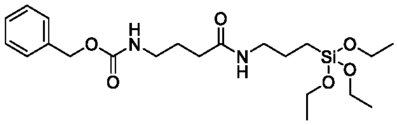 | (S)-benzyl 4-oxo-4-(3-(triethoxysilyl)propylamino)butylcarbamate |
| 9 | 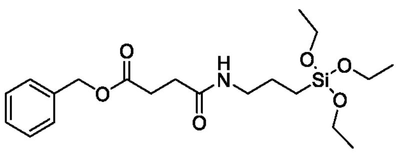 | benzyl 4-oxo-4-(3-(triethoxysilyl)propylamino)butanoate |

Production Examples

Silicon compounds were produced by the methods described below. Table 2 shows the structures and names of synthesized compounds.

Production Example 1: Synthesis of Compound (10)

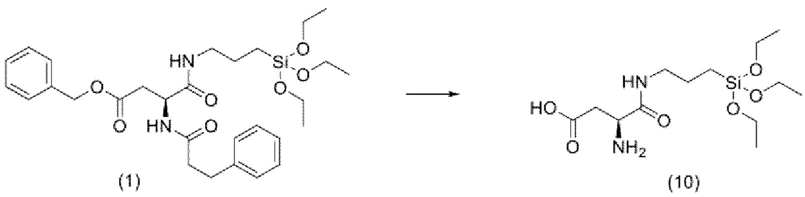

Compound (1) (980 mg) was dissolved in ethanol (20 ml), and 10% Pd-C (containing 55% H2O) (218 mg) was added, followed by hydrogenation at room temperature. After 2 hours, the disappearance of the starting material was confirmed by TLC, followed by filtration with Celite and then concentration, thereby obtaining compound (10) (539 mg) as a colorless amorphous substance.
$^1$H NMR (CD3CD2OD, 400 MHz) δ 0.60 (m, 2 H), 1.20 (t, J = 7.0 Hz, 9 H), 1.61 (m, 2 H), 2.51 (dd, J = 16.8 Hz, 9.8 Hz, 1 H), 2.64 (dd, J = 16.8 Hz, 4.7 Hz, 1 H), 3.20 (t, J = 7.2 Hz, 2 H), 3.81 (q, J

Production Example 2: Synthesis of Compound (11)

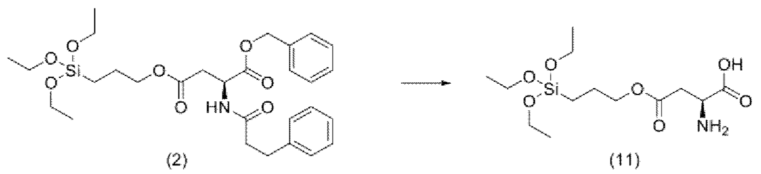

Compound (2) (645 mg) was dissolved in ethanol (15 ml), and 10% Pd-C (containing 55% H2O) (130 mg) was added, followed by hydrogenation at room temperature. After 2 hours, the disappearance of the starting material was confirmed by TLC, followed by filtration with Celite and then concentration, thereby obtaining compound (11) (368 mg) as a colorless amorphous substance.
$^1$H NMR (CD3CD2OD, 400 MHz) δ 0.61 (m, 2 H), 1.20 (t, J = 7.0 Hz, 9 H), 1.61 (m, 2 H), 2.60 (dd, J = 16.4 Hz, 9.8 Hz, 1 H), 2.92 (dd, J = 16.4 Hz, 3.4 Hz, 1 H), 3.16 (m, 2 H), 3.73 (dd, J = 9.8 Hz, 3.4 Hz, 1H), 3.81 (q, J = 7.0 Hz, 6 H).

Production Example 3: Synthesis of Compound (12)

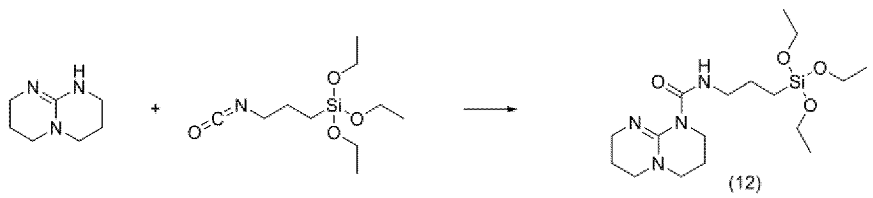

1,5,7-Triazabicyclo[4.4.0]dec-5-ene (1000 mg) was dissolved in dichloromethane (20 ml), and 3-(triethoxysilyl) propyl isocyanate (1.96 ml) was added dropwise at room temperature. After stirring for 1 hour, the reaction mixture was purified using a florisil column (eluent: $CH_2Cl_2$) to obtain compound (12) (1580 mg) as a colorless oil.
$^1$H NMR ($CDCl_3$, 400 MHz) δ 0.60-0.68 (m, 2 H), 1.22 (t, J = 7.0 Hz, 9 H), 1.58-1.68 (m, 2 H), 1.79-1.96 (m, 4 H), 3.11-3.25 (m, 6H), 3.38 (t, J = 5.6 Hz, 2 H), 3.78-3.85 (m, 8H).

Production Example 4: Synthesis of Compound (13)

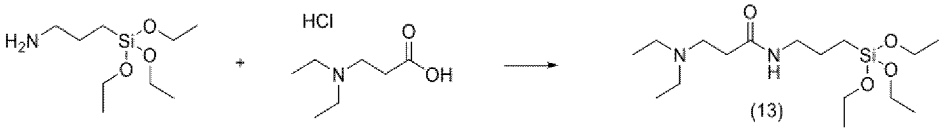

(13)

3-Aminopropyltriethoxysilane (942 mg), 3-(diethylamino)propionic acid hydrochloride (773 mg), and HOBt (32.6 mg) were dissolved in dichloromethane (10 ml), and 1-(3-dimethylaminopropyl)-3-ethyl-carbodiimide (727 mg) was added, followed by stirring at room temperature for 3 hours. The reaction mixture was purified using a florisil column (eluent: $CH_2Cl_2$) to obtain compound (13) (880 mg) as a colorless oil.
$^1$H NMR ($CDCl_3$, 400 MHz) δ 0.58-0.67 (m, 2 H), 1.05 (t, J = 7.2 Hz, 6 H), 1.22 (t, J = 7.0 Hz, 9 H), 1.56-1.63 (m, 2 H), 2.34 (t, J = 5.8 Hz, 2 H), 2.55 (q, J = 7.2 Hz, 4 H), 2.66 (t, J = 7.2 Hz, 2 H), 3.18-3.25 (m, 2 H), 3.81 (q, J = 7.0 Hz, 6 H), 8.54 (br-s, 1H).

Production Example 5: Synthesis of Compound (14)

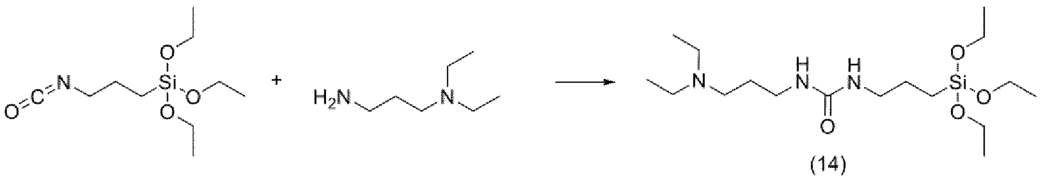

(14)

3-Diethylaminopropylamine (526 mg) was dissolved in dichloromethane (10 ml), and 3-(triethoxysilyl)propyl isocyanate (1 ml) was added dropwise, followed by stirring at room temperature for 30 minutes. The reaction mixture was concentrated under reduced pressure to obtain compound (14) (1480 mg) as a colorless oil.
$^1$H NMR ($CDCl_3$, 400 MHz) δ 0.60-0.68 (m, 2 H), 1.02 (t, J = 7.1 Hz, 6 H), 1.22 (t, J = 7.0 Hz, 9 H), 1.57-1.70 (m, 4 H), 2.45-2.57 (m, 6 H), 3.13 (q, J = 7.0 Hz, 2 H), 3.24 (t, J = 6.2 Hz, 2 H), 3.81 (q, J = 7.0 Hz, 6 H), 4.75 (br-s, 1 H), 5.53 (br-s, 1H).

Production Example 6: Synthesis of Compound (15)

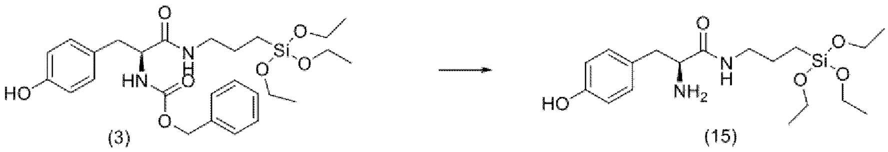

(3) (15)

Compound (3) (300 mg) was dissolved in ethanol (10 ml), and 10% Pd-C (containing 55% H2O) (30 mg) was added, followed by hydrogenation at room temperature. After reaction for 3 hours, the disappearance of the starting material was confirmed by TLC. The reaction mixture was filtered with Celite, and the filtrate was concentrated to obtain compound (15) (168 mg) as a colorless oil.
$^1$H NMR ($CDCl_3$, 400 MHz) δ 0.60-0.68 (m, 2 H), 1.22 (t, J = 7.0 Hz, 9 H), 1.50-1.64 (m, 2 H), 2.64 (dd, J = 13.8 Hz, 9.0 Hz, 1 H), 3.14 (dd, J = 13.8 Hz, 4.2 Hz, 1 H), 3.25 (td, J = 13.4 Hz, 6.9 Hz, 2 H), 3.54 (dd, J = 9.0 Hz, 4.2 Hz, 2 H), 3. 82 (q, J = 7.0 Hz, 6 H), 6.81 (d, J = 8.5 Hz, 2 H), 7.05 (d, J = 8.5 Hz, 2 H), 7d, 2 H).

Production Example 7: Synthesis of Compound (16)

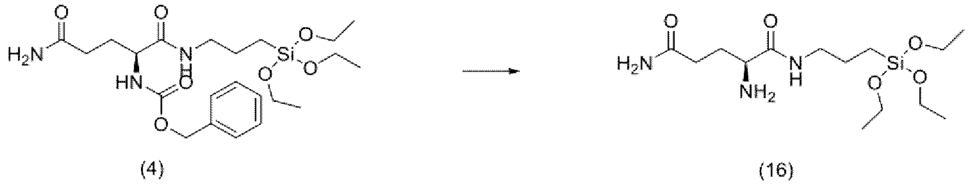

(4) (16)

Compound (4) (700 mg) was dissolved in ethanol (10 ml) and 10% Pd-C (containing 55% H2O) (77 mg) was added, followed by hydrogenation at room temperature. After reaction for 1 hour, the disappearance of the starting material was confirmed by TLC. The reaction mixture was filtered with Celite, and the filtrate was concentrated. The obtained crude product was purified using a florisil column (eluent: CH2Cl2:EtOH = 20:1) to obtain compound (16) (310 mg) as a white solid.

$^1$H NMR ($CDCl_3$, 400 MHz) δ 0.60-0.68 (m, 2 H), 1.23 (t, J = 7.0 Hz, 9 H), 1.45-1.87 (m, 6 H), 1.90-2.07 (m, 2 H), 2.37 (td, J = 6.8 Hz, 2.1 Hz, 2H), 3.19-3.30 (m, 2H), 3.44 (t, J = 6.6 Hz, 1H), 3.82 (q, J = 7.0 Hz, 6 H), 5.53 (br-s, 1 H), 6.37 (br-s, 1 H), 7.42 (br-s, 1 H).

Production Example 8: Synthesis of Compound (17)

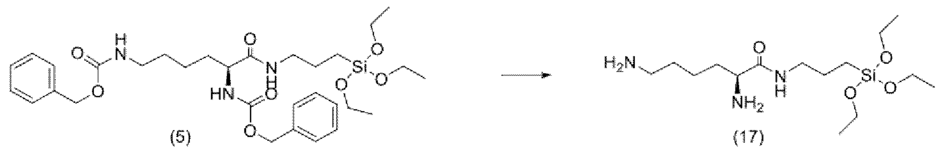

(5) (17)

Compound (5) (550 mg) was dissolved in ethanol (20 ml), and 10% Pd-C (containing 55% H2O) (95 mg) was added, followed by hydrogenation at room temperature. After reaction for 3 hours, the disappearance of the starting material was confirmed by TLC. The reaction mixture was filtered with Celite, and the filtrate was concentrated. The obtained crude product was purified using a florisil column (eluent: CH2Cl2:EtOH = 20:1) to obtain compound (17) (161 mg) as a colorless oil.

$^1$H NMR ($CDCl_3$, 400 MHz) δ 0.63 (br-t, J = 8.2 Hz, 2 H), 1.22 (t, J = 7.0 Hz, 9 H), 1.31-1.57 (m, 8 H), 1.57-1.68 (m, 3 H), 1.78-1.90 (m, 1 H), 2.70 (t, J = 6.8 Hz, 2H), 3.25 (q, J = 6.9 Hz, 2 H), 3.31-3.37 (m, 1 H), 3.82 (q, J = 7.0 Hz, 6 H), 7.27 (br-s, 1 H).

Production Example 9: Synthesis of Compound (18)

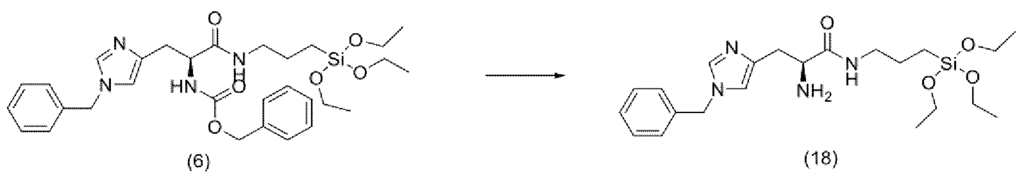

(6) (18)

Compound (6) (1400 mg) was dissolved in ethanol (10 ml), and 10% Pd-C (containing 55% H2O) (128 mg) was added, followed by hydrogenation at room temperature. After reaction for 5 hours, the disappearance of the starting material was confirmed by TLC. The reaction mixture was filtered with Celite, and the filtrate was concentrated. The obtained crude product was purified using a florisil column (eluent: CH2Cl2:EtOH = 20:1) to obtain compound (18) (470 mg) as a colorless oil.

$^1$H NMR ($CDCl_3$, 400 MHz) δ 0.58-0.68 (m, 2 H), 1.22 (t, J = 7.0 Hz, 9 H), 1.57 (tt, J = 7.8 Hz, 7.2 Hz, 2 H), 1.65-1.95 (m, 2 H), 2.74-2.82 (m, 1 H), 3.03 (dt, J = 4.5 Hz, 1.7 Hz, 1 H), 3.13-3.26 (m, 2 H), 3.56-3.61 (m, 1H), 3.81 (q, J = 7.0H

Production Example 10: Synthesis of Compound (19)

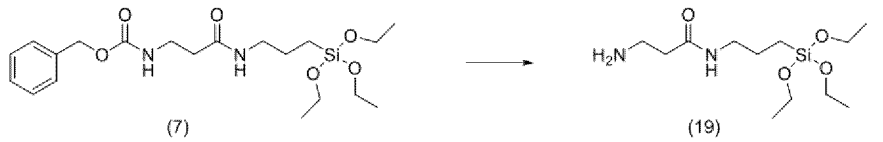

(7) (19)

Compound (7) (1100 mg) was dissolved in ethanol (20 ml), and 10% Pd-C (containing 55% H2O) (183 mg) was added, followed by hydrogenation at room temperature. After reaction for 2 hours, the disappearance of the starting material was confirmed by TLC. The reaction mixture was filtered with Celite, and the filtrate was concentrated. The obtained crude product was purified using a florisil column (eluent: CH2Cl2:EtOH = 20:1) to obtain compound (19) (285 mg) as a colorless oil.

$^1$H NMR ($CDCl_3$, 400 MHz) δ 0.62-0.68 (m, 2 H), 1.23 (t, J = 7.0 Hz, 9 H), 1.47 (br-s, 2 H), 1.58-1.68 (m, 2 H), 2.31 (t, J = 6.0 Hz, 2 H), 3.00 (dd, J = 6.1 Hz, 5.9 Hz, 2 H), 3.26 (dd, J = 12.9 Hz, 7.0 Hz, 2 H), 3.82 (q, J = 7.0 Hz, 6 H), 7.28 (br-s, 1 H). z, 6 H), 5.04 (s, 2 H), 7.15 (d, J = 7.3 Hz, 2 H), 7.28-7.38 (m, 3 H), 7.44 (s, 1 H), 7.48 (br-t, J = 5.5 Hz, 1 H).

Production Example 11: Synthesis of Compound (20)

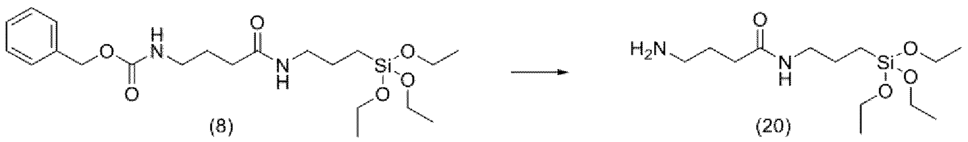

(8) (20)

Compound (8) (1600 mg) was dissolved in ethanol (25 ml), and 10% Pd-C (containing 55% H2O) (193 mg) was added, followed by hydrogenation at room temperature. After reaction for 3 hours, the disappearance of the starting material was confirmed by TLC. The reaction mixture was filtered with Celite, and the filtrate was concentrated. The obtained crude product was purified using a florisil column (eluent: CH2Cl2:EtOH = 10:1) to obtain compound (20) (870 mg) as a colorless oil.

$^1$H NMR (CDCl3, 400 MHz) δ 0.60-0.68 (m, 2 H), 1.23 (t, J = 7.0 Hz, 9 H), 1.58-1.67 (m, 4 H), 1.77 (tt, J = 7.4 Hz, 6.9 Hz, 2 H), 2.23 (t, J = 7.3 Hz, 2 H), 2.36 (t, J = 6.3 Hz, 2 H), 2743 (t, J = 6.8 Hz, 2 H), 3.14-3.21 (m, 2 H), 3.82 (q, J = 7.0 Hz, 6 H), 5.05-5.34 (m, 2 H), 6.05 (br-s, 1 H).

Production Example 12: Synthesis of Compound (21)

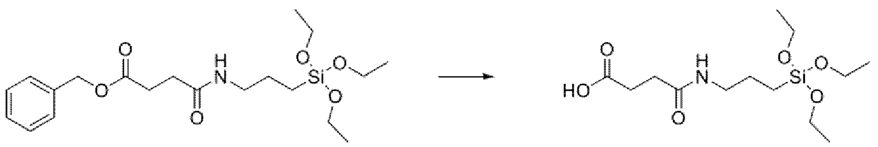

(9) (21)

Compound (9) (590 mg) was dissolved in ethanol (20 ml), and 10% Pd-C (containing 55% H2O) (153 mg) was added, followed by hydrogenation at room temperature. After reaction for 3 hours, the disappearance of the starting material was confirmed by TLC. The reaction mixture was filtered with Celite to remove the catalyst, and the filtrate was then concentrated to obtain compound (21) (270 mg) as a colorless oil.

$^1$H NMR (CDCl3, 400 MHz) δ 0.60-0.68 (m, 2 H), 1.23 (t, J = 7.0 Hz, 9 H), 1.58-1.67 (m, 4 H), 1.77 (tt, J = 7.4 Hz, 6.9 Hz, 2 H), 2.23 (t, J = 7.3 Hz, 2 H), 2.36 (t, J = 6.3 Hz, 2 H), 2743 (t, J = 6.8 Hz, 2 H), 3.14-3.21 (m, 2 H), 3.82 (q, J = 7.0 Hz, 6 H), 5.05-5.34 (m, 2 H), 6.05 (br-s, 1 H).

Table 2

| Table 2 | | |
|---|---|---|
| Compound | STR | Name of compound* |
| 10 | 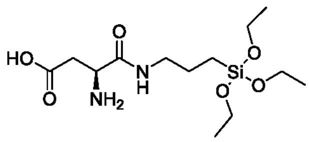 | (S)-3-amino-4-oxo-4-(3-(triethoxysilyl)propylamino)butanoic acid |
| 11 | 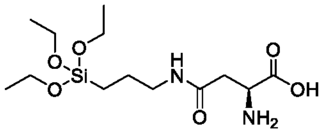 | (R)-3-amino-4-oxo-4-(3-(triethoxysilyl)propylamino)butanoic acid |
| 12 | 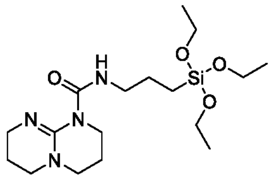 | N-(3-(triethoxysilyl)propyl)-2,3,4,6,7,8-hexahydro 1H-pyrido[1,2-a]pyrimidine-1-carboxamide |
| 13 | 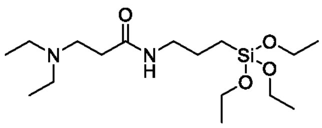 | 3-(diethylamino)-N-(triethoxysilyl)propyl)propanamide |
| 14 | 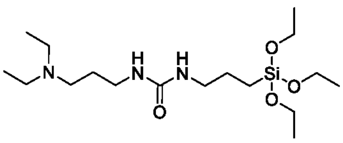 | 1-(3-(diethylamino)propyl)-3-(triethoxysilyl)propyl)urea |
| 15 | 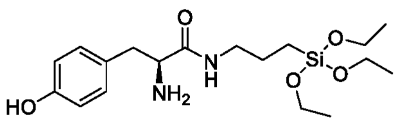 | (S)-2-amino-3-(4-hydroxyphenyl)-N-(3-(triethoxysilyl)propyl)propanamide |
| 16 | 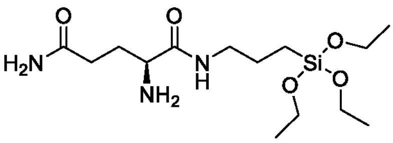 | (S)-2-amino-N-1-(3-(triethoxysilyl)propyl)pentanediamide |
| 17 | 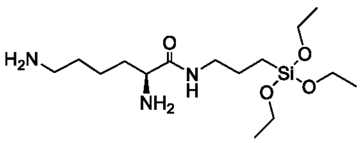 | (S)-2,6-diamino-N-(3-(triethoxysilyl)propyl)hexanam ide |
| 18 | 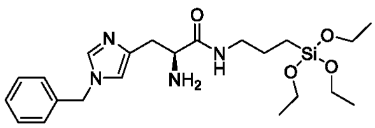 | (S)-2-amino-3-(1-benzyl-1H-imidazol-4-yl)-N-(3-(triethoxysilyl)propyl)propanamide |

Examples

Example 1: Production of Cetylpyridinium Chloride Hydrate-containing Silicon Nanoparticle Composition using Compounds (S1) and (S2)

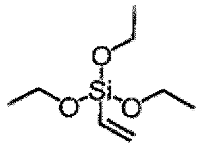
(S1)

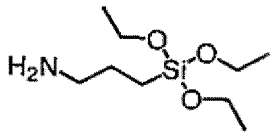
(S2)

1-Butanol (0.8 ml) and dioctyl sodium sulfosuccinate (440 mg) were dissolved in pure water (20 ml) to prepare solution A. Next, cetylpyridinium chloride hydrate (37 mg) was dissolved in N,N-dimethylformamide (1 ml) to prepare solution B. Solution A, solution B, and compound (S1) (0.2 ml; produced by Tokyo Chemical Industry Co., Ltd.) were added to a round-bottom flask (volume: 50 ml) and stirred at room temperature for 1 hour. Subsequently, compound (S2) (0.1 ml; produced by Tokyo Chemical Industry Co., Ltd.) was added, followed by stirring for another hour. The resulting mixture was dialyzed with a dialysis membrane (Float-A-Lyzer (registered trademark) G2 produced by Spectrum Laboratories, molecular weight cut-off (MWCO): 20 kD) for 48 hours. The dialyzed solution was sterile-filtered through a 0.22 μm filter (syringe filter produced by Techno Lab SC (product number: TLMC25022)).

Example 2: Production of Cetylpyridinium Chloride Hydrate-containing Silicon Nanoparticle Composition using Compound (S3)

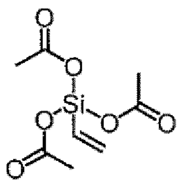
(S3)

Solution A (5.2 ml) and solution B (0.25 ml) described in Example 1, and compound (S3) (47.6 μl; produced by Sigma-Aldrich) were added to a sample tube (volume: 30 ml) and stirred at room temperature for 2 hours. The resulting mixture was dialyzed with a dialysis membrane (Float-A-Lyzer (registered trademark) G2 produced by Spectrum Laboratories; molecular weight cut-off (MWCO): 20 kD) for 48 hours. The dialyzed solution was sterile-filtered through a 0.22 μm filter (syringe filter produced by Techno Lab SC (product number: TLMC25022)).

Examples 3 to 10

The following cetylpyridinium chloride hydrate-containing silicon nanoparticle compositions of Examples 3 to 9 were produced in the same manner as in Example 1, except that the following silicon compounds (S4) to (S10) were used in place of compound (S1).

Table 3 shows the silicon compounds used in Examples 3 to 10.

Table 3

| Example | Silicon compound | Example | Silicon compound |
|---|---|---|---|
| 3 | 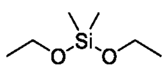 (S4) | 7 | 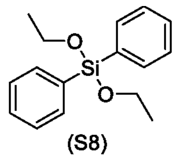 (S8) |
| 4 | 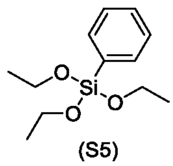 (S5) | 8 | 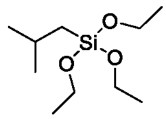 (S9) |

(continued)

| Example | Silicon compound | Example | Silicon compound |
|---|---|---|---|
| 5 | 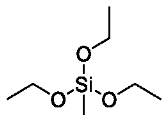 (S6) | 9 | 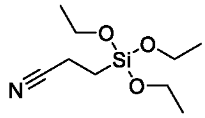 (S10) |
| 6 | 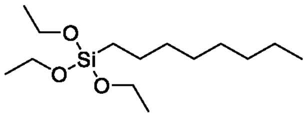 (S7) | 10 | 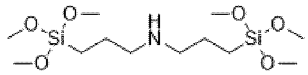 (S11) |

Example 11: Production of Cetylpyridinium Chloride Hydrate-containing Silicon Nanoparticle Composition using Compounds (S3) and (S2)

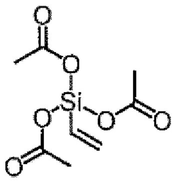

(S3)

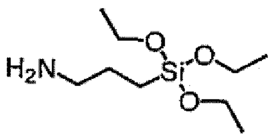

(S2)

Solution A (5.2 ml) and solution B (0.25 ml) described in Example 1, and compound (S3) (47.6 μl; produced by Sigma-Aldrich) were added to a sample tube (volume: 30 ml) and stirred at room temperature for 1 hour. Compound (S2) (25 μl; produced by Tokyo Chemical Industry Co., Ltd.) was then added, followed by stirring for another hour. The resulting mixture was dialyzed with a dialysis membrane (Float-A-Lyzer (registered trademark) G2 produced by Spectrum Laboratories; molecular weight cut-off (MWCO): 20 kD) for 48 hours. The dialyzed solution was sterile-filtered through a 0.22 μm filter (syringe filter produced by Techno Lab SC (product number: TLMC25022)).

Example 12: Production of Cetylpyridinium Chloride Hydrate-containing Silicon Nanoparticle Composition using Compounds (S3) and (10)

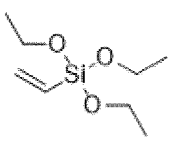

(S1)

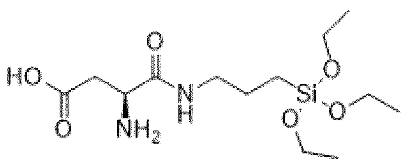

(10)

Solution A (5.2 ml) and solution B (0.25 ml) described in Example 1, and compound (S1) (50 μL) were added to a sample tube (volume: 30 mL) and stirred at room temperature for 1 hour. Compound (10) (40 mg) was then added, followed by stirring for another hour. The resulting mixture was dialyzed with a dialysis membrane (Float-A-Lyzer (registered trademark) G2 produced by Spectrum Laboratories; MWCO: 20 kD) for 48 hours. The dialyzed solution was sterile-filtered through a 0.22 μm filter (Durapore and Millex, both of which are registered trademarks, produced by Merck Millipore).

Example 13: Production of Cetylpyridinium Chloride Hydrate-containing Silicon Nanoparticle Composition using Compounds (S3) and (11)

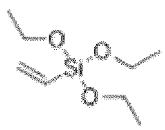

(S1)

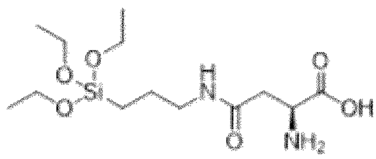

(1 1)

Solution A (5.2 ml) and solution B (0.25 ml) described in Production Example 1, and compound (S1) (50 μL) were added to a sample tube (volume: 30 mL) and stirred at room temperature for 1 hour. Compound (11) (40 mg) was then added, followed by stirring for another hour. The resulting mixture was dialyzed with a dialysis membrane (Float-A-Lyzer (registered trademark) G2 produced by Spectrum Laboratories; MWCO: 20 kD) for 48 hours. The dialyzed solution was sterile-filtered through a 0.22 μm filter (Durapore and Millex, both of which are registered trademarks, produced by Merck Millipore).

The drug concentration, drug loading rate, and average particle size of each of the cetylpyridinium chloride hydrate-containing silicon nanoparticle compositions produced in Examples 1 to 11 were measured. Table 4 shows the results.

Table 4

| Example | Cetylpyridinium chloride concentration (μg/ml) (a) | Cetylpyridinium chloride concentration (%) (b) | Average particle size (nm) (c) |
|---|---|---|---|
| 1 | 1233.0 | 98.7 | 55.98 |
| 2 | 1334.1 | 93.4 | 222.6 |
| 3 | 1294.3 | 97.5 | 97.94 |
| 4 | 1079.9 | 96.6 | 113.7 |
| 5 | 1324.8 | 98.3 | 69.96 |
| 6 | 721.2 | 98.0 | 123.7 |
| 7 | 657.4 | 92.8 | 155.4 |
| 8 | 577.4 | 99.3 | 129.5 |
| 9 | 1122.2 | 94.9 | 132.9 |
| 11 | 630.9 | 99.6 | 129.8 |
| 12 | 1187.7 | 76.1 | 215.9 |
| 13 | 376.9 | 93.0 | 201.6 |

The cetylpyridinium chloride concentration was measured by HPLC. (a) Each cetylpyridinium chloride hydrate-containing silicon nanoparticle composition was subjected to ultrafiltration using an ultrafiltration membrane with a MWCO of 300 K (Pall). The concentration of cetylpyridinium chloride in the ultrafiltrate was measured by HPLC to determine the amount of unloaded cetylpyridinium chloride.
The loading rate was calculated by the following formula.

$$\text{Drug loading rate (\%)} = \frac{\text{Drug peak area in suspension} - \text{Drug peak area in 300 Da ultrafiltrate}}{\text{Drug peak area in suspension}} \times 100$$

The average particle size was measured as the effective diameter by dynamic light scattering using a Zetasizer Nano ZS (produced by Malvern).

Next, the method for producing a drug-containing silica nanoparticle composition is described with reference to

Experimental Examples (antimicrobial agent).

Experimental Examples

Experimental Example 1: Production of Levofloxacin-containing Silicon Nanoparticle Composition (LVF-NPs)

1-Butanol (0.8 ml) and dioctyl sodium sulfosuccinate (440 mg) were dissolved in pure water (20 ml) to prepare solution A. Next, levofloxacin (37.4 mg) was dissolved in N,N-dimethylformamide (1 ml) to prepare solution B. Solution A, solution B, and triethoxyvinylsilane (0.2 ml; produced by Tokyo Chemical Industry Co., Ltd.) were added to a round-bottom flask (volume: 50 ml) and stirred at room temperature for 1 hour. Subsequently, (3-aminopropyl)triethoxysilane (0.1 ml; produced by Tokyo Chemical Industry Co., Ltd.) was added, followed by stirring for another hour. The resulting mixture was dialyzed with a dialysis membrane (Float-A-Lyzer (registered trademark) G2 produced by Spectrum Laboratories; molecular weight cut-off (MWCO): 20 kD) for 48 hours. The dialyzed solution was sterile-filtered through a 0.22 μm filter (syringe filter produced by Techno Lab SC (product number: TLMC25022)) to obtain the title composition.

Experimental Example 2: Production of Ciprofloxacin-containing Silica Nanoparticle Composition (CPFX-NPs)

1-Butanol (0.8 ml) and dioctyl sodium sulfosuccinate (440 mg) were dissolved in pure water (20 ml) to prepare solution A. Next, ciprofloxacin (34.3 mg) was dissolved in N,N-dimethylformamide (1 ml) to prepare solution B. Solution A, solution B, and triethoxyvinylsilane (0.2 ml; produced by Tokyo Chemical Industry Co., Ltd.) were added to a round-bottom flask (volume: 50 ml) and stirred at room temperature for 1 hour. Subsequently, (3-aminopropyl)triethoxysilane (0.1 ml; produced by Tokyo Chemical Industry Co., Ltd.) was added, followed by stirring for another hour. The resulting mixture was dialyzed with a dialysis membrane (Float-A-Lyzer (registered trademark) G2 produced by Spectrum Laboratories; molecular weight cut-off (MWCO): 20 kD) for 48 hours. The dialyzed solution was sterile-filtered through a 0.22 μm filter (syringe filter produced by Techno Lab SC (product number: TLMC25022)) to obtain the title composition.

Experimental Example 3: Production of Clinafloxacin-containing Silica Nanoparticle Composition (CLFX-NPs)

1-Butanol (0.8 ml) and dioctyl sodium sulfosuccinate (440 mg) were dissolved in pure water (20 ml) to prepare solution A. Next, clinafloxacin (37.9 mg) was dissolved in N,N-dimethylformamide (1 ml) to prepare solution B. Solution A, solution B, and triethoxyvinylsilane (0.2 ml; produced by Tokyo Chemical Industry Co., Ltd.) were added to a round-bottom flask (volume: 50 ml) and stirred at room temperature for 1 hour. Subsequently, (3-aminopropyl)triethoxysilane (0.1 ml; produced by Tokyo Chemical Industry Co., Ltd.) was added, followed by stirring for another hour. The resulting mixture was dialyzed with a dialysis membrane (Float-A-Lyzer (registered trademark) G2 produced by Spectrum Laboratories; molecular weight cut-off (MWCO): 20 kD) for 48 hours. The dialyzed solution was sterile-filtered through a 0.22 μm filter (syringe filter produced by Techno Lab SC (product number: TLMC25022)) to obtain the title composition.

Explanation of Efficacy

Efficacy 1

Cetylpyridinium Chloride Hydrate-containing Silicon Nanoparticle Composition Produced in Example

Antimicrobial Activity Evaluation of Example 1

Test purpose: evaluation of antimicrobial activity of Example 1 against the following test strains
Test strain: *S. aureus* FDA 209P (MSSA), *E. coli* ATCC 8739, *S. aureus* MU-3 (MRSA), *P. aeruginosa* ATCC 27853
Test method: broth microdilution method according to Clinical Laboratory Standards Institute

(i) Each strain stored at -80°C is applied to MHII agar and cultured at 35°C for 16 to 20 hours.
(ii) The cultured bacteria are suspended in MHIIB to McFarland 0.5, and the suspensions are diluted 100-fold and adjusted to a bacterial count of about $10^6$ CFU/mL with MHIIB.
(iii) Each compound stock solution is prepared with MHIIB to the top dose shown in the next section, and 2-fold dilution is repeated with MHIIB to prepare compound-containing media.
(iv) Each compound-containing medium in (iii) is dispensed in an amount of 50 μL into a 96-well plate, followed by inoculation with 50 μL of each bacterial liquid in (ii). Culture is performed at 35°C for 20 to 24 hours.

Determination method: The lowest concentration at which no bacterial growth is observed with the naked eye is regarded as the minimum inhibitory concentration.

Table 5

| | MIC (μg/mL) | | | |
|---|---|---|---|---|
| | *S. aureus* FDA 209P | *S. aureus* MU3 | *E. coli* ATCC 8739 | *P. aeruginosa* ATCC 27853 |
| CPC* | 5 | 32 | 200 | >1000 |
| Example 1 | 10 | 64 | >200 | >1000 |
| *CPC: cetylpyridinium chloride hydrate | | | | |

Efficacy 2

Evaluation of Antimicrobial Activity of Coated Material

Test purpose: Evaluation of Presence or Absence of Antimicrobial Activity of Cetylpyridinium Chloride Hydrate-containing Silicon Nanoparticle Composition (ACPC-NPs) Produced in Example 1 Coated on Various Materials
Test strain: *S. aureus* FDA 209P (MSSA), *E. coli* ATCC 8739, ***S.*** *aureus* MU-3 (MRSA), *P. aeruginosa* ATCC 27853
Test method:

(i) Each strain stored at -80°C is applied to MHII agar and cultured at 35°C for 16 to 20 hours.

(ii) The cultured bacteria are suspended in MHIIB to McFarland 0.5, followed by suitable dilution with MHIIB to a bacterial count of about 104 CFU/ml.

(iii) 0.2 mL of each of the resulting test bacterial liquids is individually added dropwise on MHII agar, and each test specimen (50 mm × 50 mm) is individually placed thereon. The petri dish (in which the bacterial liquid spreads naturally due to the weight of the test specimen) is covered, followed by culture at 35°C for 20 to 24 hours.

Determination method: It is determined that it is effective when colony growth is clearly inhibited compared with the untreated test specimen.

Table 6

| Sample | Coated material | Amount of coating that completely suppresses colony growth (μg/cm$^2$) | | | |
|---|---|---|---|---|---|
| | | *S. aureus* FDA 209P | *S. aureus* MU3 | *E. coli* ATCC 8739 | *P. aeruginosa* ATCC 27853 |
| Example 1 | Titanium | < 16.4 | 65.6 | > 65.6 | > 65.6 |
| | Ceramic | < 16.4 | > 65.6 | > 65.6 | > 65.6 |

Efficacy 3

The drug elution from materials coated with the nanoparticle compositions produced in Experimental Examples 1 to 3 and Example 1 was evaluated.

Test conditions

Material: titanium metal (5 × 5 cm)
Amount of drug coated: 100 μg/25 cm$^2$ (4 μg/cm$^2$)
Processing method: after dropwise addition, air drying

Test vessel: glass petri dish
Test liquid: PBS pH 7.4, 20 ml
Shaking conditions: 37°C, 10 rpm
Sampling point: 1 hr, 2 hr, 4 hr, 8 hr, 24 hr
NPs samples evaluated:

Table 7

| | Drug concentration |
|---|---|
| Experimental Example 1 (LVFX-NPs) | 592.0 μg/ml |
| Experimental Example 2 (CPFX-NPs) | 505.2 μg/ml |
| Experimental Example 3 (CLFX-NPs) | 1175.8 μg/ml |
| Example 1 | 1783.9 μg/ml |

Drug elution rate (%)

Table 8

| | Sampling point | | | | |
|---|---|---|---|---|---|
| Drug | 1 hr | 2 hr | 4 hr | 8 hr | 24 hr |
| Experimental Example 1 | 100% | 100% | 100% | 100% | 100% |
| Experimental Example 2 | 80% | 100% | 100% | 100% | 100% |
| Experimental Example 3 | 95% | 100% | 100% | 100% | 100% |
| Example 1 | 0% | 0% | 0% | 0% | 0% |

Efficacy 4

The sustained antimicrobial activity of the cetylpyridinium chloride hydrate-containing silicon nanoparticle composition (ACPC-NPs) produced in Example 1 was evaluated by the following experiment.

Using an untreated titanium plate (1) and a titanium plate coated with the cetylpyridinium chloride hydrate-containing silicon nanoparticle composition (2), the sustained antimicrobial activity was evaluated according to the following test method, using the growth of methicillin-resistant *Staphylococcus aureus* MRSA (*S. aureus* MU3) on each test specimen as an index.

Test method:

(i) One test specimen (1) (untreated titanium plate; 50 mm × 50 mm) and one test specimen (2) (titanium plate coated with cetylpyridinium chloride hydrate-containing silicon nanoparticle composition of Example 1; 50 mm × 50 mm) are placed in separate 9 cm petri dishes, and 20 ml of PBS is added, followed by shaking at 37°C and 55 rpm.
(ii) The PBS is replaced with fresh PBS every day. On 1, 3, 5, 7, 14, 21, and 28 days after the start of shaking, each test specimen is taken out, the surface is rinsed with PBS, and then the test specimens are allowed to dry naturally and used for evaluation of antimicrobial activity.
(iii) *S. aureus* MU-3 strain stored at -80°C is applied to Mueller Hinton II agar (MHIIA) and cultured at 35°C for 16 to 20 hours. Thereafter, the cultured bacteria are suspended in Muller Hinton II broth (MHIIB) to a McFarland turbidity of 0.5, followed by suitable dilution to a bacterial count of about 104 CFU (colony forming unit)/ml (test bacterial liquid).
(iv) 0.2 ml of the test bacterial liquid is added dropwise onto MHIIA, and each test specimen is individually placed thereon and covered, followed by culture at 35°C for 20 to 24 hours.
(v) The growth of the bacteria in test specimen (1) is considered as +++, and the growth of the bacteria in test specimen (2) is evaluated as +++ when it is similar to that in test specimen (1), ++ when it is generally 50% or less of that in test specimen (1), + when the number of colonies is generally several to 50 or less, and - when the growth of the bacteria is not observed. The cases of - to ++ are determined as having antimicrobial activity.

Test results

As shown in Table 9, the growth of the bacteria was observed in test specimen (1) from the first day of the test. In contrast, no growth of the bacteria was observed in test specimen (2) even 14 days after the start of the test. Although slight growth of the bacteria was observed on day 21, the growth was still slight on day 28.

This test showed sustained antimicrobial activity on the surface of the titanium plate by coating with the cetylpyridinium chloride hydrate-containing silicon nanoparticle composition.

Table 9

| Table 7: Evaluation of antimicrobial activity on surface of titanium plate | | | | | | | | |
|---|---|---|---|---|---|---|---|---|
| | Day 0 | Day 1 | Day 3 | Day 5 | Day 7 | Day 14 | Day 21 | Day 28 |
| | Inoculation amount of *S. aureus* MU-3 (CFU/plate) | | | | | | | |
| Sample | $2.94 \times 10^3$ | $2.94 \times 10^3$ | $3.66 \times 10^3$ | $3.42 \times 10^3$ | $3.02 \times 10^3$ | $4.32 \times 10^3$ | $2.00 \times 10^3$ | $2.00 \times 10^3$ |
| Test specimen (1) | +++ | +++ | +++ | +++ | +++ | +++ | +++ | +++ |
| Test specimen (2) | - | - | - | - | - | - | + | + |

Efficacy 5

Antiviral Activity Evaluation Test of Cetylpyridinium Chloride Hydrate-containing Silicon Nanoparticle Composition Produced in Example 1

This test was conducted at the Kobe testing center of the Japan Textile Products Quality and Technology Center.

Test conditions:

Test virus: influenza virus A

Test sample:

(i) 0.06% cetylpyridinium chloride hydrate-containing silicon nanoparticle composition aqueous solution (Example 1)

(ii) 0.006% cetylpyridinium chloride hydrate-containing silicon nanoparticle composition aqueous solution (1/10 concentration of Example 1)

Test conditions:

virus suspension:test sample = 1:9
Action temperature: 25°C
Action time: 15 seconds, 5 minutes Infectivity titer measurement method: plaque measurement method

Test results

Table 10

| Material sample | | Common logarithm average value of virus infectivity titer (PFU/mL) | Difference in common logarithm value from purified water (blank) (PFU/mL) | Virus reduction compared with purified water (blank) |
|---|---|---|---|---|
| Purified water (blank) | | 7.10 | -- | |
| 0.06% Example 1 aqueous solution | After 15 seconds of action | 2.22 | 4.9 | 99.999% |
| | After 5 minutes of action | < 2.0 | 5.1 | 99.999% |
| Purified water (blank) | | 7.36 | - | |
| 0.006% Example 1 aqueous solution | After 15 seconds of action | 6.73 | 0.6 | 23.4% |
| | After 5 minutes of action | 2.81 | 4.5 | 99.999% |

Industrial Applicability

The present invention provides a nanoparticle composition comprising cetylpyridinium chloride hydrate and a silicon compound that exhibits a sustained antiviral effect and a sustained antimicrobial or bactericidal effect. In addition, the present invention can be used in a drug delivery system for preventing and/or treating an infectious disease using a medical material or medical device or an orthopedic medical device carrying the composition.

Description of the Reference Numerals

10, 100: Inactivation filter device
20, 200: Layer of filter member
30: Layer of nanoparticles
40, 50: Moisture-permeable waterproof layer
60: Tank
64: Aqueous layer
66: Visualization means

**Claims**

1. A composition in the form of nanoparticles, comprising cetylpyridinium chloride hydrate and a specific silicon compound represented by the following formula (I):

Formula (I)

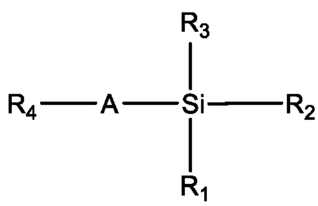

wherein

A represents a bond or lower alkylene;
$R_1$, $R_2$, and $R_3$ each independently represent

(1) lower alkyl,
(2) O-$R_5$, or
(3) phenyl;

$R_5$ represents

(1) lower alkyl or
(2) lower alkanoyl;

$R_4$ represents

(1) lower alkyl,
(2) lower alkenyl,
(3) phenyl,
(4) amino optionally substituted with one to two identical or different $R_6$s,
(5) $R_6$-CONH, or
(6) cyano;

$R_6$ represents

(1) lower alkyl optionally substituted with one to two identical or different $R_7$s,
(2) amino optionally substituted with one to two identical or different $R_8$s, or
(3) silyl lower alkyl optionally substituted with one to three identical or different lower alkoxy groups;

$R_7$ represents

(1) amino optionally substituted with one to two identical or different lower alkyl groups,
(2) phenyl optionally substituted with hydroxy,
(3) carbamoyl,
(4) imidazolyl optionally substituted with benzyl,
(5) carboxy, or
(6) guanidino optionally substituted with benzyloxycarbonyl; and

$R_8$ represents

(1) amino lower alkyl, wherein the amino is optionally substituted with one to two identical or different lower alkyl groups, or
(2) carboxy.

**2.** The composition according to claim 1, wherein the compound of formula (I) is a compound in which

A represents a bond or $C_{1-6}$ alkylene;
$R_1$, $R_2$, and $R_3$ each independently represent

(1) $C_{1-6}$ alkyl,
(2) O-$R_5$, or
(3) phenyl;

$R_5$ represents

(1) $C_{1-6}$ alkyl or
(2) $C_{1-6}$ alkanoyl;

$R_4$ represents

(1) $C_{1-8}$ alkyl,
(2) $C_{2-4}$ alkenyl,
(3) phenyl,
(4) amino optionally substituted with one $R_8$,
(5) $R_6$-CONH, or
(6) cyano;

$R_6$ represents

(1) lower alkyl optionally substituted with one to two identical or different $R_7$s,
(2) amino optionally substituted with one to two identical or different $R_8$s,
(3) silyl $C_{1-6}$ alkyl optionally substituted with one to three identical or different lower alkoxy groups;

$R_7$ represents

(1) amino optionally substituted with one to two identical or different $C_{1-6}$ alkyl groups,
(2) phenyl substituted with hydroxy,
(3) carbamoyl,
(4) benzylimidazolyl,
(5) carboxy, or
(6) guanidino; and

$R_8$ represents amino-$C_{1-6}$ alkyl, wherein the amino is optionally substituted with one to two identical or different $C_{1-6}$ alkyl groups; or two $R_8$s together with the nitrogen to which they are attached may form a saturated or unsaturated 3- to 12-membered monocyclic or bicyclic heterocycle containing at least one nitrogen as a ring-constituting heteroatom.

**3.** The composition according to claim 1 or 2, wherein the compound of formula (I) is a compound in which

A represents $C_{1-6}$ alkylene;
$R_1$, $R_2$, and $R_3$ each independently represent O-$R_5$;
$R_5$ represents $C_{1-6}$ alkyl;
$R_4$ represents $R_6$-CONH;
$R_6$ represents

(1) $C_{1-6}$ alkyl optionally substituted with one to two identical or different $R_7$s or
(2) amino optionally substituted with one to two identical or different $R_8$s;

$R_7$ represents

(1) amino optionally substituted with two identical or different $C_{1-6}$ alkyl groups,
(2) phenyl substituted with hydroxy,
(3) carbamoyl,
(4) benzylimidazolyl,
(5) carboxy, or
(6) guanidino; and

$R_8$ represents amino-$C_{1-6}$ alkyl, wherein the amino is optionally substituted with one to two identical or different $C_{1-6}$ alkyl groups.

**4.** The composition according to claim 1, which is sterile-filterable.

**5.** The composition according to claim 1, which is carried by a preventive device and/or a medical device for use in the prevention and/or treatment of an infectious disease.

**6.** A medical material or medical device carrying the composition of claim 1.

Fig. 1

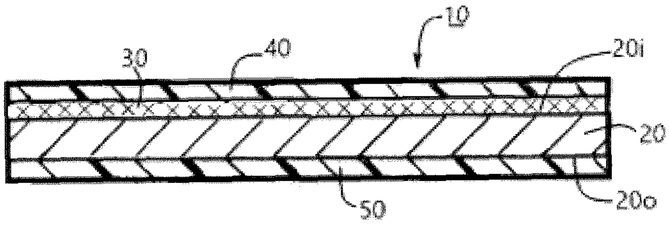


Fig. 2

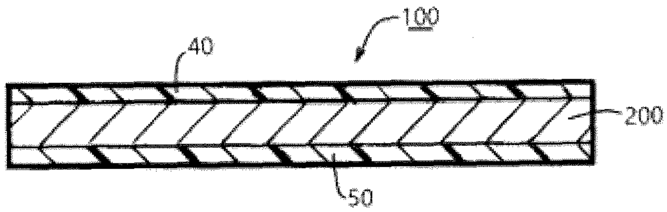


Fig. 3

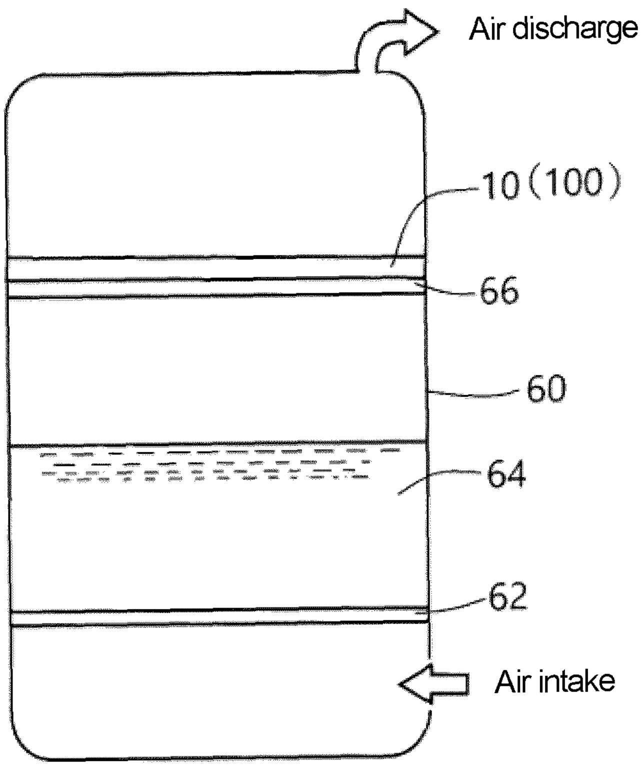

**INTERNATIONAL SEARCH REPORT**

International application No. **PCT/JP2023/028430**

**A. CLASSIFICATION OF SUBJECT MATTER**

***A61K 31/4425***(2006.01)i; ***A61K 9/14***(2006.01)i; ***A61K 9/51***(2006.01)i; ***A61K 47/24***(2006.01)i; ***A61L 9/16***(2006.01)i; ***A61L 27/28***(2006.01)i; ***A61L 27/54***(2006.01)i; ***A61L 29/08***(2006.01)i; ***A61L 29/16***(2006.01)i; ***A61L 31/08***(2006.01)i; ***A61L 31/16***(2006.01)i; ***A61P 31/00***(2006.01)i; ***B01D 39/16***(2006.01)i; ***B01D 39/18***(2006.01)i; ***D06M 13/463***(2006.01)i

FI: A61K31/4425; A61K9/14; A61K9/51; A61K47/24; A61L27/28; A61L27/54; A61L31/08; A61L31/16; A61L29/08; A61L29/16; A61P31/00; B01D39/16 Z; B01D39/18; D06M13/463; A61L9/16 F

According to International Patent Classification (IPC) or to both national classification and IPC

**B. FIELDS SEARCHED**

Minimum documentation searched (classification system followed by classification symbols)

A61K31/4425; A61K9/14; A61K9/51; A61K47/24; A61L9/16; A61L27/28; A61L27/54; A61L29/08; A61L29/16; A61L31/08; A61L31/16; A61P31/00; B01D39/16; B01D39/18; D06M13/463

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | SEO, J. Y. et al. Downstream integration of microalgae harvesting and cell disruption by means of cationic surfactant-decorated Fe3O4 nanoparticles. Green Chem. 2016, 18, pp. 3981-3989<br>entire text | 1, 2, 4-6 |
| A | entire text | 3 |
| A | KR 10-2016-0137109 A (CHA UNIVERSITY, INDUSTRY ACADEMIC COOPERATION FOUNDATION) 30 November 2016 (2016-11-30)<br>entire text | 1-6 |
| A | JP 2008-266157 A (GUNMA UNIV) 06 November 2008 (2008-11-06)<br>entire text | 1-6 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 October 2023** | **17 October 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/028430**

**C. DOCUMENTS CONSIDERED TO BE RELEVANT**

| Category* | Citation of document, with indication, where appropriate, of the relevant passages | Relevant to claim No. |
|---|---|---|
| A | ROY, I. et al. Ceramic-Based Nanoparticles Entrapping Water-Insoluble Photosensitizing Anticancer Drugs: A Novel Drug-Carrier System for Photodynamic Therapy. J. AM. CHEM. SOC. 2003, 125(26), pp. 7860-7865<br>entire text | 1-6 |
| P, A | WO 2022/208322 A1 (TOMSON TECHNOLOGIES LLC) 06 October 2022 (2022-10-06)<br>entire text | 1-6 |

Form PCT/ISA/210 (second sheet) (January 2015)

**INTERNATIONAL SEARCH REPORT**
**Information on patent family members**

International application No.
**PCT/JP2023/028430**

| Patent document cited in search report | | | Publication date (day/month/year) | Patent family member(s) | Publication date (day/month/year) |
|---|---|---|---|---|---|
| KR | 10-2016-0137109 | A | 30 November 2016 | (Family: none) | |
| JP | 2008-266157 | A | 06 November 2008 | (Family: none) | |
| WO | 2022/208322 | A1 | 06 October 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007505697 A
- JP 2008266157 A
- JP 2008506493 A
- JP 2012533568 A

### Non-patent literature cited in the description


- **C. XIE** ; **H. ZAO** ; **K. LI** ; **Z. ZHANG** ; **X. LU** ; **H. PANG** ; **D. WANG** ; **J. CHEN** ; **X. ZHANG** ; **D. WU**. The evidence of indirect transmission of SARS-CoV-2 reported in Guangzhou. *China BMC Public Health*, 2020, vol. 20, 1202
- **S. H. BAE** ; **H. SHIN** ; **H-Y. KOO** ; **S. W. LEE** ; **J. M. YANG** ; **D. K. YON**. Asymptomatic transmission of SARS-Co-2 on evacuation flight. *Emerg. Infect, Dis.*, 2020, vol. 26, 2705
- *Journal of American Chemical Society*, 2003, vol. 125 (26), 7860-7865
- Organic Functional Group Preparation. Academic Press, Inc., 1989
- Comprehensive Organic Transformations. VCH Publishers Inc., 1989
- **P. G. M. WUTS** ; **T. W. GREENE**. Greene's Protective Groups in Organic Synthesis. 2006